# EUROPEAN PATENT SPECIFICATION

(11) **EP 1 370 275 B1**
(45) Date of publication and mention of the grant of the patent: **26.05.2010**
(21) Application number: 02733865.6
(22) Date of filing: 19.03.2002
(51) Int. Cl.: A61K 31/7032, A61K 31/485, A61K 38/33, A61P 25/04

(54) **METHODS FOR INCREASING ANALGESIC POTENCY AND ATTENUATING ADVERSE EXCITATORY EFFECTS OF BIMODALLY-ACTING OPIOID AGONISTS BY INHIBITING GM1-GANGLIOSIDE**
VERFAHREN ZUR ERHÖHUNG DER ANALGETISCHEN WIRKUNG UND ZUR VERRINGERUNG DER UNERWÜNSCHTEN EXITATORISCHEN WIRKUNGEN VON BIMODALISCH WIRKSAMEN OPIOIDAGONISTEN DURCH DIE HEMMUNG VON GM1-GANGLIOSID
PROCEDES PERMETTANT D'AUGMENTER L'ACTIVITE ANALGESIQUE ET D'ATTENUER LES EFFETS EXCITATEURS INDESIRABLES D'AGONISTES OPIOIDES AGISSANT DE FACON BIMODALE PAR INHIBITION DU GANGLIOSIDE GM1

(30) Priority: 23.03.2001 US 278529 P
(43) Date of publication of application: 17.12.2003
(73) Proprietor: ALBERT EINSTEIN COLLEGE OF MEDICINE OF YESHIVA UNIVERSITY, Bronx New York 10461 (US)
(72) Inventor: CRAIN, Stanley, M., State College, PA 16803 (US); SHEN, Ke-Fei, Flushing, NY 11355 (US)
(74) Representative: Gowshall, Jonathan Vallance
(86) International application number: PCT/US2002/008472
(87) International publication number: WO 2002/076388

(56) References cited:
- US-A- 5 364 842
- US-B1- 6 174 878

## Description

### Background of the Invention

Morphine and many other opioid agonists have analgesic effects that are mediated by their activation of inhibitory opioid receptors on nociceptive (pain-mediating) neurons (9). Accordingly, these opioids are administered to relieve severe pain. Morphine and many other opioid agonists, however, also have been shown to activate excitatory opioid receptors on nociceptive neurons, thereby attenuating the analgesic potency of the opioid agonists, and resulting in the development of anti-analgesia, hyperexcitability, hyperalgesia, physical dependence, psychological dependence, tolerance, and other adverse (excitatory) effects (9, 10, 12, 14, 15, 48, 51, 52). Consequently, a long-standing need has existed to develop a method which will both enhance the analgesic (inhibitory) effects of these bimodally-acting opioid agonists and block or prevent adverse (excitatory) effects associated with their administration. The present invention satisfies this need.

Previous patents have disclosed that the analgesic potency of bimodally-acting opioid agonists can be enhanced, and the tolerance/dependence liability reduced, by co-administering the bimodally-acting opioid agonists with ultralow doses of selective excitatory opioid receptor antagonists (*e.g*., U.S. Patent Nos. 5,472,943; 5,512,578; 5,580,876; and 5,767,125). Excitatory opioid receptor antagonists are compounds that bind to and inactivate excitatory opioid receptors, but not inhibitory opioid receptors, on neurons in nociceptive (pain) pathways. Selective excitatory opioid receptor antagonists attenuate excitatory, but not inhibitory, opioid receptor functions in nociceptive pathways of the peripheral and central nervous systems. As a result, symptoms associated with activation of excitatory opioid receptors (*e.g.*, anti-analgesia, hyperalgesia, hyperexcitability, physical dependence, and tolerance effects) are blocked, while the analgesic effects of the bimodally-acting opioid agonists, which are mediated by the inhibitory opioid receptors, are unmasked and thereby enhanced (10, 11, 12, 52).

Previous patents have further disclosed that ultralow doses of naltrexone, alone or in combination with low-dose methadone (*e.g.*, U.S. Patent No. 5,512,578), and ultralow doses of other excitatory opioid receptor antagonists alone (*e.g.*, U.S. Patent Nos. 5,580,876 and 5,767,125), can provide effective, long-term maintenance treatment for opioid addiction after acute detoxification, and can prevent relapse to drug abuse. Furthermore, preclinical studies have suggested that ultralow doses of selective excitatory opioid receptor antagonists can be administered alone to chronic pain patients to enhance the analgesic potency and reduce the tolerance/dependence liability of endogenous opioid peptides, such as enkephalins, dynorphins, and endorphins, which are elevated in chronic pain patients (10).

GM1 is a monosialoganglioside that is abundantly distributed on the external surface of neuronal cell membranes (23, 24, 25, 37, 62). GM1-ganglioside plays a role in regulating excitatory opioid receptors in nociceptive neurons, probably by binding to an allosteric regulatory site on opioid receptors (11, 50, 53). Previous studies (67, 68) and earlier patents (*e*.*g*., U.S. Patent Nos. 5,321,012; 5,502,058; 5,556,838; and 5,654,281) have suggested that the analgesic potency of opioid agonists is enhanced, and the tolerance/dependence liability of endogenous opioid peptides is reduced, when opioids are co-administered with high doses of exogenous GM1-ganglioside. More recent evidence has demonstrated that intraperitoneal (i.p.) injection of low doses of exogenous GM1-ganglioside (0.1 mg/kg) in mice rapidly attenuates morphine's analgesic effects (13).

Cholera toxin consists of a non-covalently-assembled pentamer of a non-toxic B subunit (CTX-B) that is responsible for cell attachment, and a toxicogenic A subunit (CTX-A) (39). CTX-B binds selectively to GM1-ganglioside on the surface of cells, and facilitates penetration of CTX-A into the cell membrane (23, 27, 43). CTX-A selectively catalyzes the ADP-ribosylation of Gₛ (28, 40), resulting in inhibition of an associated GTPase (6), increased adenylate cyclase activity, and decreased efficacy of ligand-activation of Gₛ-coupled receptors (5, 6, 38, 41, 55).

Previous studies have shown that pretreatment of dorsal root ganglion (DRG) neurons with CTX-B selectively blocks opioid-induced prolongation of the action potential duration (APD), but not opioid-induced shortening of the APD (50), suggesting that GM1-ganglioside may regulate Gₛ-linked excitatory opioid receptor functions in DRG neurons. Additional studies on DRG neurons in culture have shown that chronic opioid treatment, together with administration of nanomolar concentrations of CTX-B, prevents development of tolerance to the inhibitory, APD-shortening effects of micromolar concentrations of the opioid, by preventing the development of opioid excitatory supersensitivity - a cellular manifestation related to opioid tolerance and dependence *in vivo* (51).

While CTX-A has been shown to block opioid excitatory effects by ADP-ribosylation of Gₛ (49), CTX-B blockade appears to involve interference with GM1-ganglioside regulation of opioid excitatory receptor functions. In particular, it is known that CTX-B binds with selective high affinity (K_{D} = 10⁻¹⁰M) to GM1-ganglioside (23, 24, 25, 37, 62). Treatment of DRG neurons with anti-GM1 antibodies has been shown selectively to block opioid-induced APD prolongation, as occurs with CTX-B (50).

### Summary of the Invention

The present invention is predicated on the surprising discovery that the analgesic potency of morphine - a bimodally-acting opioid agonist - is increased when morphine is co-administered in mice with CTX-B or oseltamivir - agents that inhibit specific functions of endogenous GM1-ganglioside on opioid receptors in nociceptive neurons. This discovery was unexpected, as the prior art suggested that administration of high doses of exogenous GM1-ganglioside increased the analgesic potency of opioid agonists, and attenuated morphine tolerance and dependence (67, 68). On the basis of this finding, the present invention provides a method for increasing analgesic potency of a bimodally-acting opioid agonist in a subject, by administering to the subject an analgesic or subanalgesic amount of a bimodally-acting opioid agonist, in combination with an amount of an agent that inhibits GM1-ganglioside in nociceptive neurons effective to increase the analgesic potency of the bimodally-acting opioid agonist.

Also provided by the present invention is the use for the manufacture of a medicament for treating pain in a subject in need of treatment thereof, by administering to the subject a bimodally-acting opioid agonist in combination with an agent that inhibits GM1-ganglioside in nociceptive neurons, in amounts effective to treat the pain in the subject.

Additionally, the present invention provides a method for treating adverse excitatory effects associated with administration of a bimodally-acting opioid agonist in a subject in need of treatment thereof, by administering to the subject an agent that inhibits GM1-ganglioside in nociceptive neurons in an amount effective to treat the adverse excitatory effects in the subject.

Finally, the present invention provides a pharmaceutical composition, comprising a pharmaceutically-acceptable carrier, an analgesic or subanalgesic amount of a bimodally-acting opioid agonist, and an amount of an agent that inhibits GM1-ganglioside in nociceptive neurons effective to increase the analgesic potency of the bimodally-acting opioid agonist in a subject to whom the composition is administered.

Additional objects of the present invention will be apparent in view of the description which follows.

### Brief Description of the Figures

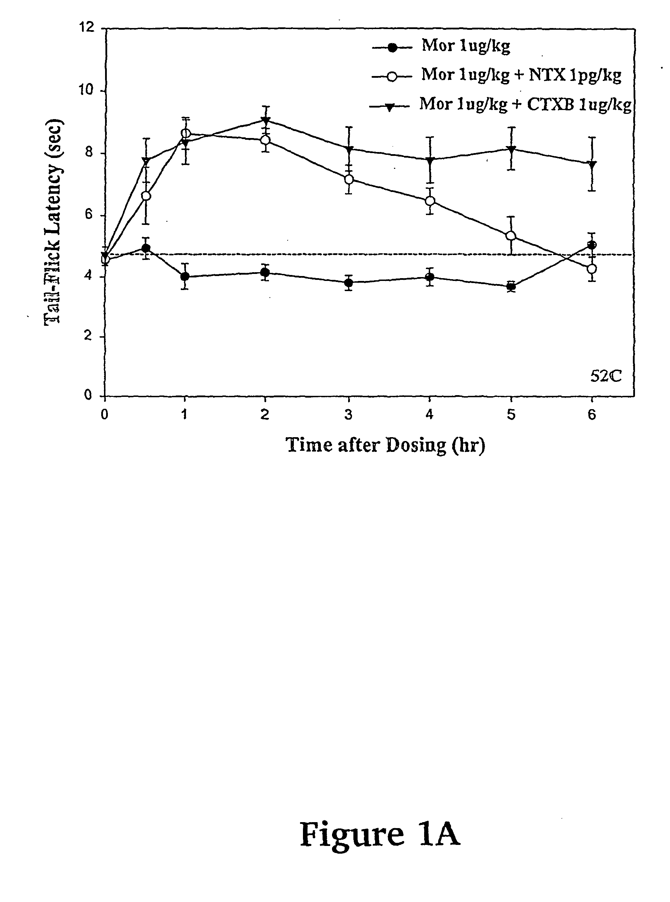

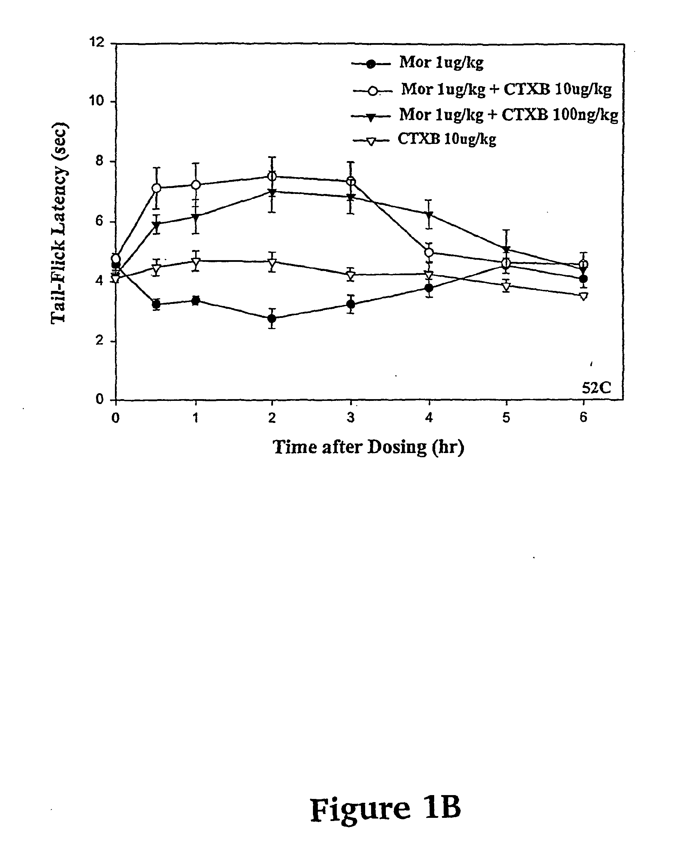

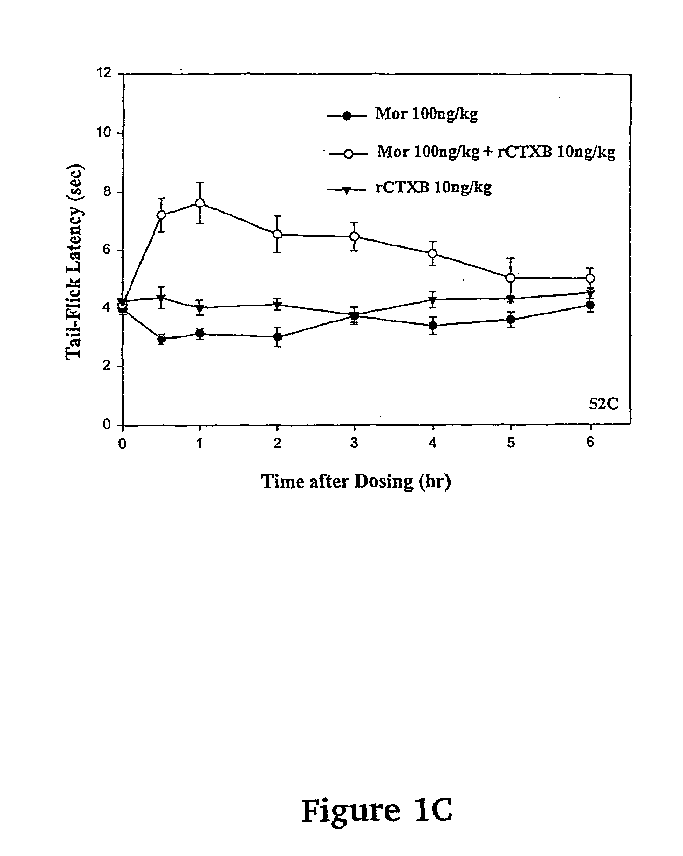

Figure 1 shows that cotreatment of mice with cholera toxin B subunit (CTXB) blocks acute, low-dose, morphine-induced thermal hyperalgesia, unmasking potent opioid analgesia. Presented are time-effect curves of hot-water-immersion (52°C) tail-flick tests. A: Administration of 1 µg/kg morphine (Mor) (s.c.) resulted in onset of decreases in tail-flick latencies, which lasted for 4-5 h after drug injection (●). Cotreatment with 1 µg/kg morphine plus 1 µg/kg CTXB (s.c.) blocked this low-dose, morphine-induced thermal hyperalgesia, and unmasked potent opioid analgesia that lasted for > 6 h (▼). This effect was similar to that produced by cotreatment with 1 pg/kg naltrexone (NTX) (○). The dashed line has been inserted to facilitate visualization of the hyperalgesic effects. B: Cotreatment with a still lower dose of CTXB (0.1 µg/kg) was also effective (▼; *cf*. 1 µg/kg morphine alone). Control tests showed that 10 µg/kg CTXB alone was ineffective (∇; *cf*. 1 µg/kg Mor + 10 µg/kg CTX-B:●). C: Cotreatment with recombinant CTX-B (rCTXB) at an even lower dose (10 ng/kg) also blocked low-dose (100 ng/kg), morphine-induced hyperalgesia, thereby unmasking potent opioid analgesia (○; *cf*. ●). All mice used were male. n=8 for each curve; error bars indicate S.E.M.

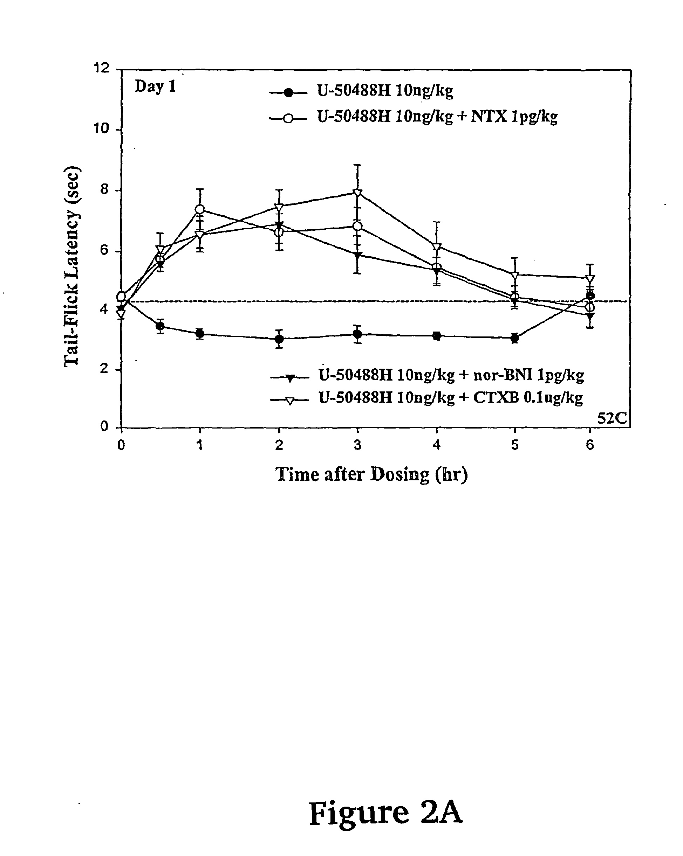

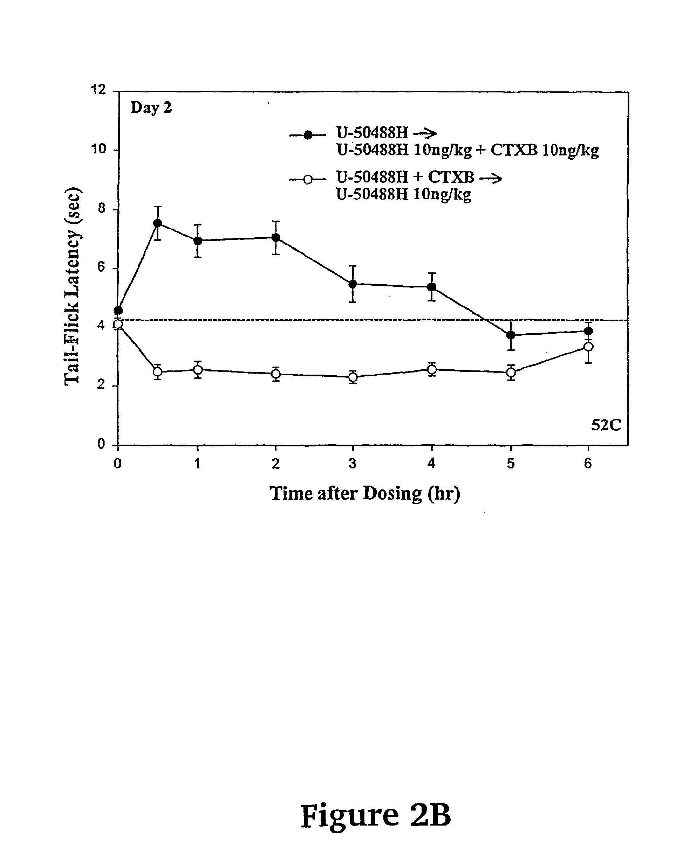

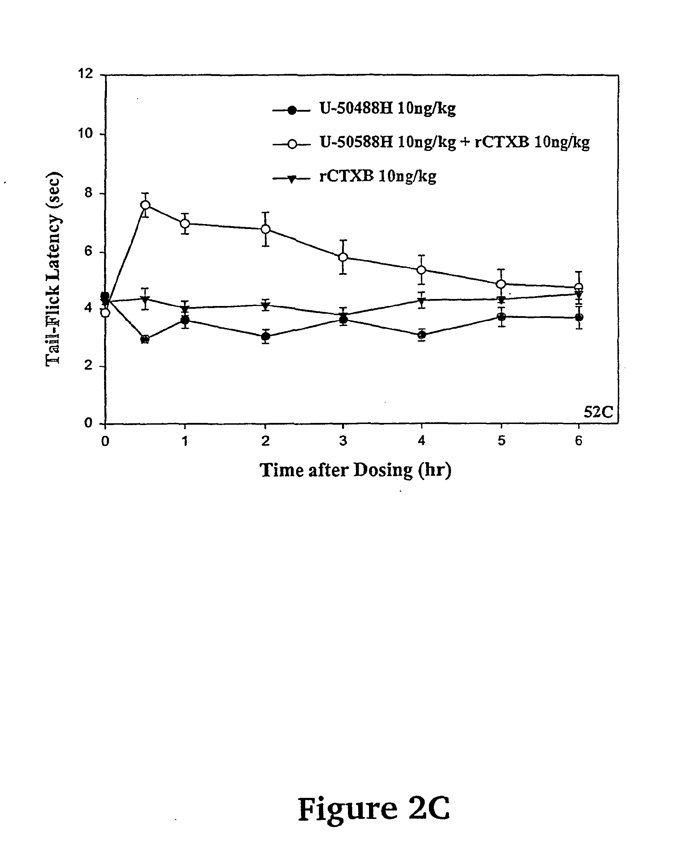

Figure 2 illustrates that cotreatment with cholera toxin B subunit (CTXB) blocks acute, low-dose, k-opioid-induced hyperalgesia, thereby unmasking potent analgesia. A: Administration of a low dose of the κ-opioid agonist, U-50,488H (10 ng/kg; s.c.), resulted in hyperalgesia similar to that elicited by low doses of the µ-opioid agonist, morphine (●; *cf*. Fig. 1A:●). Cotreatment with U-50,488H plus 0.1 µg/kg CTX-B blocked this low-dose, κ-opioid hyperalgesia, and unmasked potent analgesia (∇), with effects similar to those produced by cotreatment with an ultralow dose of either naltrexone (NTX) (○) or the specific κ-opioid antagonist, nor-binaltorphimine (nor-BNI) (▼). The dashed line has been inserted to facilitate visualization of the hyperalgesic effects. B: The group of mice that showed prominent analgesia following cotreatment with U-50,488H plus CTXB (A:∇) was retested after 24 h by treatment with U-50,488H alone. Typical hyperalgesia occurred (○), indicating the disappearance of the CTXB blocking effect. Similarly, the group of mice that showed hyperalgesia following treatment with U-50,488H alone (A:●) was retested after 24 h by cotreatment with a remarkably low dose of CTXB (10 ng/kg). In the result, the hyperalgesia was blocked, thereby unmasking prominent analgesia (●). The dashed line has been inserted to facilitate visualization of the hyperalgesic effects. C: Cotreatment of another group of mice with 10 ng/kg recombinant CTXB (rCTXB) plus 10 ng/kg U-50,488H also blocked acute κ-opioid-induced hyperalgesia (●), and unmasked potent analgesia (○), as occurred with regular CTXB (B:●). Treatment with rCTXB alone was ineffective (▼). All mice used were male. n=8 for each curve; error bars indicate S.E.M.

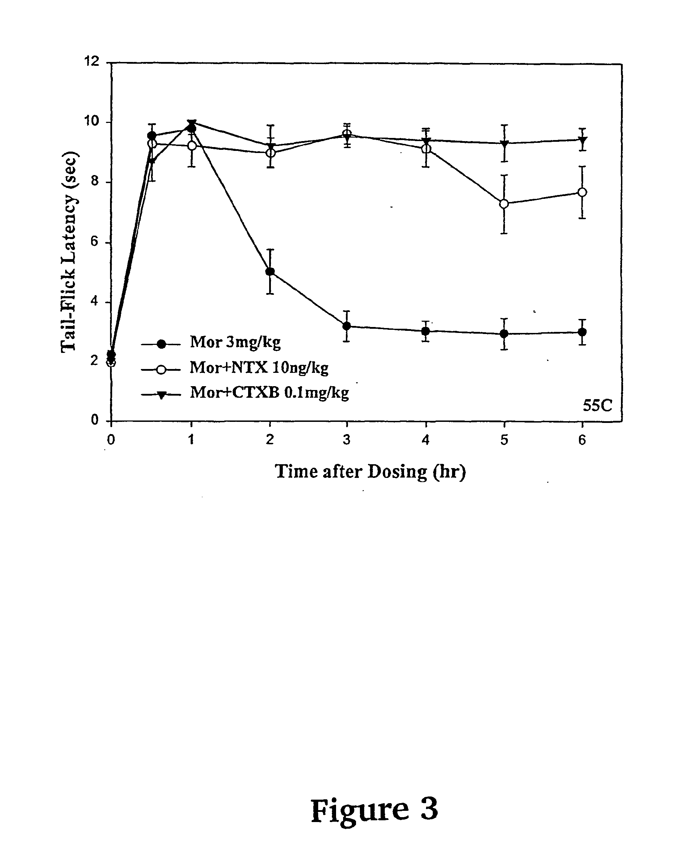

Figure 3 shows that acute cotreatment of mice with CTX-B markedly prolongs the analgesic effects of higher doses of morphine (Mor). Cotreatment with 0.1 mg/kg cholera toxin B (CTXB) (s.c.) resulted in marked prolongation of the peak antinociceptive effect of 3 mg/kg morphine for >5 h (▼), whereas the effect of morphine alone sharply decreased after the first-hour peak (●). The tail-flick assays were carried out using a slightly higher water-immersion temperature (55°C), resulting in control tail-flick latencies (ca. 2 sec) that were shorter than those obtained (4 sec) in assays at 52°C (Figs. 1 and 2). All mice used were male. n=8 for each curve; error bars indicate S.E.M.; NTX=naltrexone

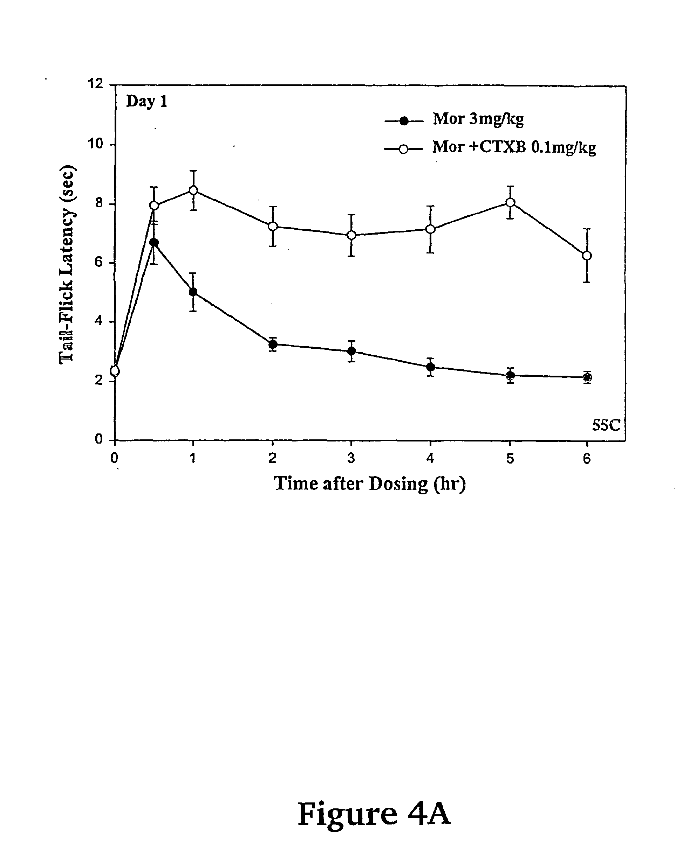

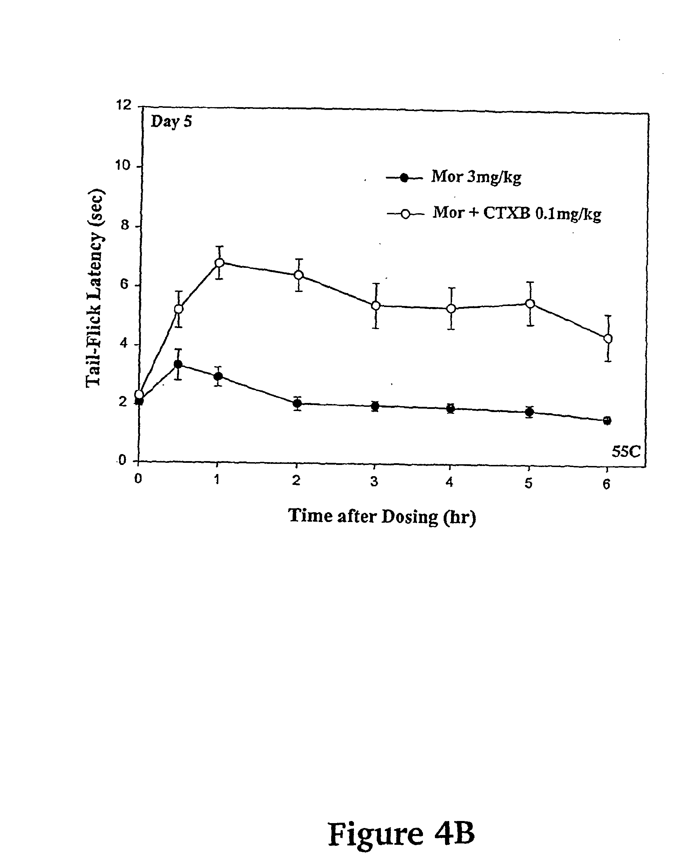

Figure 4 illustrates that chronic cotreatment of mice with morphine (Mor) plus cholera toxin B subunit (CTXB) blocks the development of opioid tolerance. A: Acute cotreatment of another group of mice with 0.1 mg/kg CTXB (s.c.) increased the magnitude as well as the duration of the antinociceptive effects of 3 mg/kg morphine (●; *cf.* Fig. 3). B: After daily injections of 3 mg/kg morphine plus CTXB, the magnitude and duration of morphine's antinociceptive effects were still quite large (○). By contrast, the analgesic effects of morphine alone were sharply decreased by 5 days (●). The tail-flick assays were carried out using a slightly higher water-immersion temperature (55°C), resulting in control tail-flick latencies (ca. 2 sec) that were shorter than those obtained (4 sec) in assays at 52°C (Figs. 1 and 2). All mice used were male. n=8 for each curve; error bars indicate S.E.M.

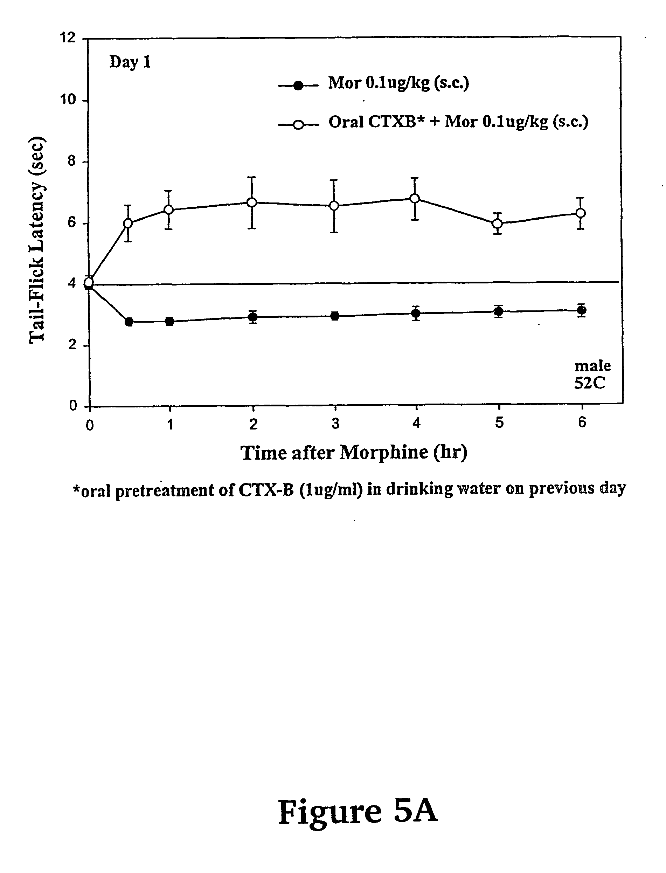

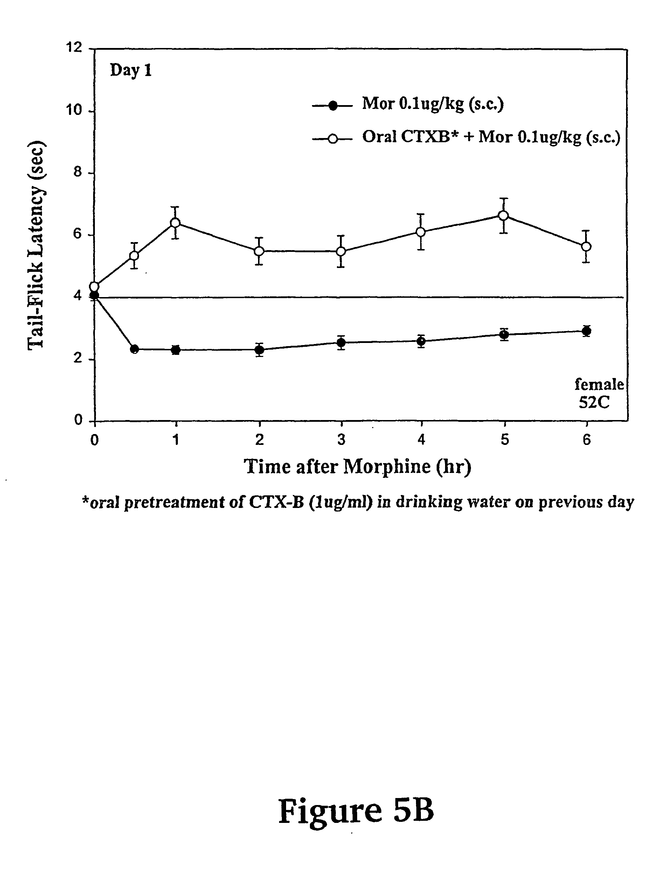

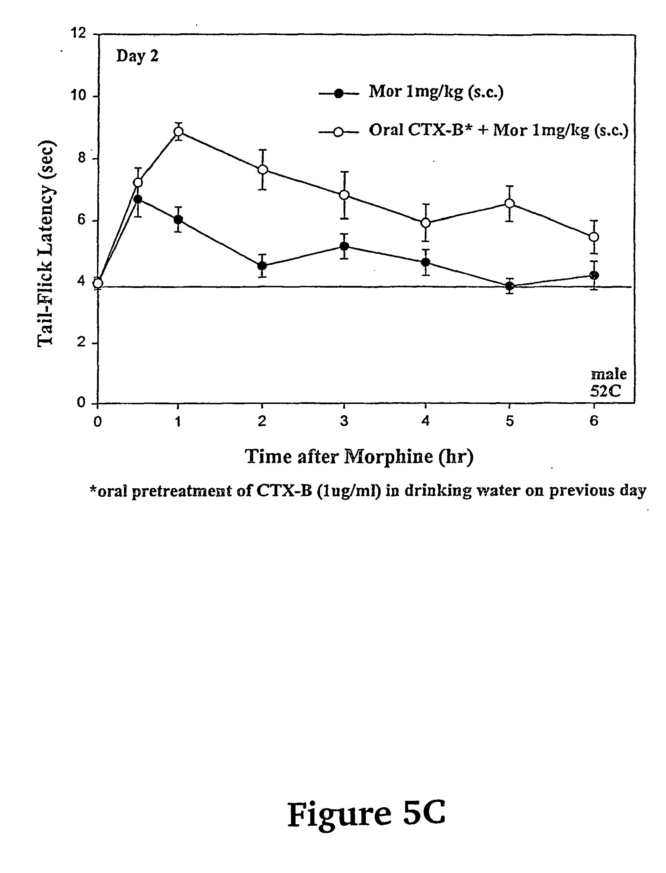

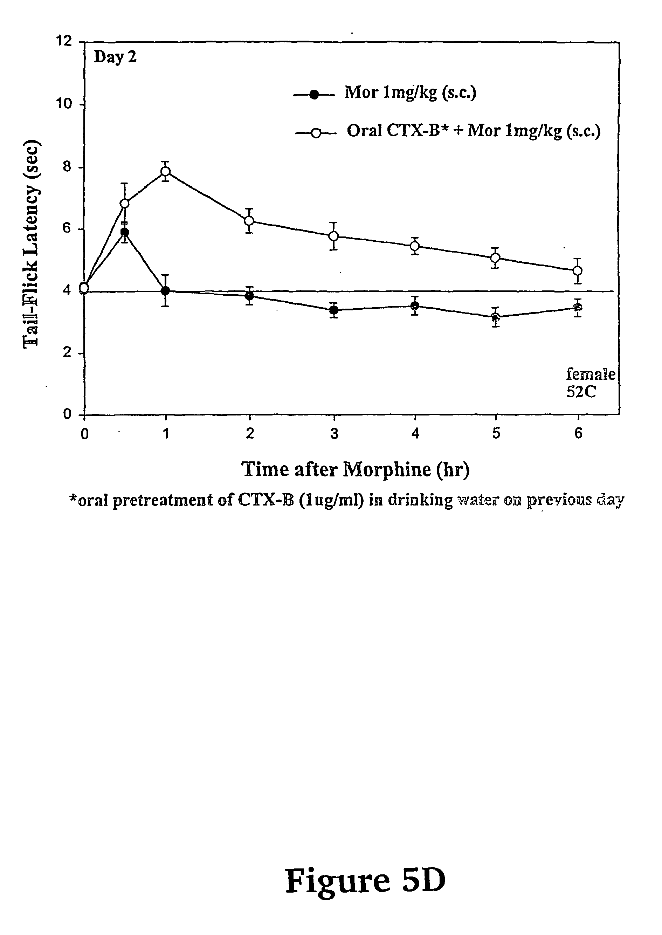

Figure 5 shows that cotreatment of male and female mice with orally-administered cholera toxin B subunit (CTXB) blocks acute, low-dose, morphine-induced hyperalgesic effects, thereby unmasking potent opioid analgesia. Tail-flick tests were performed at 52°C, as in Figs. 1 and 2. A: Administration of 0.1 µg/kg morphine (Mor) (s.c.) resulted in characteristic hyperalgesia (●), as in Fig. 1:●. In contrast, after oral pretreatment of another group of mice with CTXB (added a day earlier to the drinking-water bottles, at a concentration of 1 µg/ml), low-dose, morphine-induced hyperalgesia was blocked, and prominent opioid analgesia was unmasked (○). B: The protocol that was used in A also was carried out on a group of female mice, resulting in a similar demonstration of oral-CTXB blockade of morphine-induced hyperalgesia and a similar unmasking of opioid analgesia (○). C, D: After a second day of oral-CTXB treatment, the same groups of male and female mice were assayed by testing the effect of a 10,000-fold increase in acute morphine dose (1 mg/kg; s.c.). Much larger increases in the magnitude and duration of morphine's antinociceptive effects were noted in CTXB-treated mice (○). Although morphine's analgesic effects in the control group of female mice were considerably weaker, resulting in hyperalgesia by 2 h after opioid injection (●), the CTXB-treated group showed prominent morphine analgesia during the entire test period (○). n=8 for each curve; error bars indicate S.E.M.; *=oral pretreatment of CTXB (1 µg/ml) in drinking water on previous day

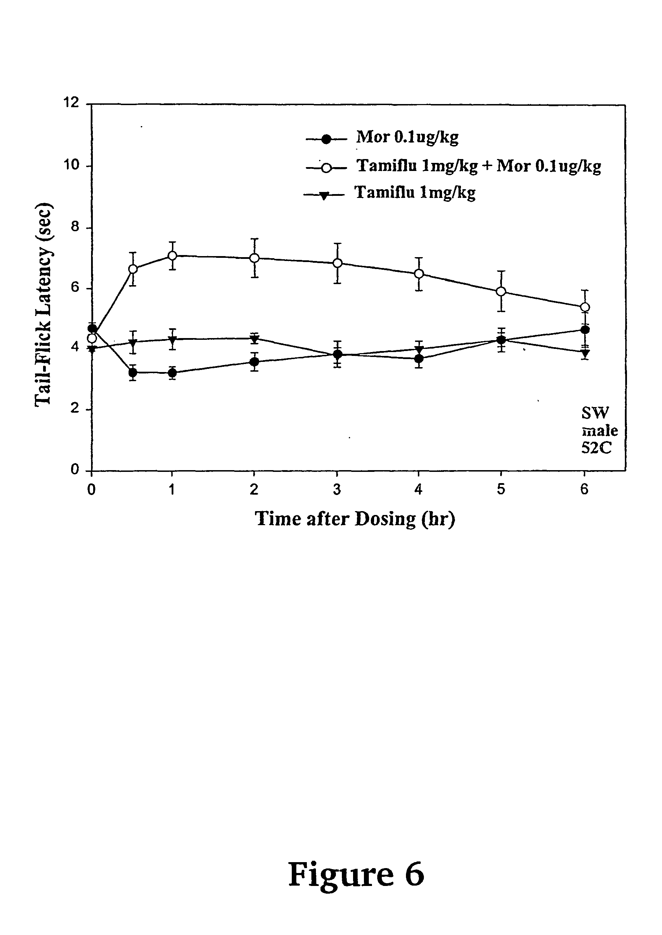

Figure 6 illustrates that cotreatment of male mice with morphine (Mor) and the neuraminidase inhibitor, oseltamivir (Tamiflu), blocks low-dose, morphine-induced hyperalgesia (●), and unmasks potent opioid analgesia (○) in hot-water-immersion, tail-flick antinociception assays. All drugs were injected subcutaneously (13).

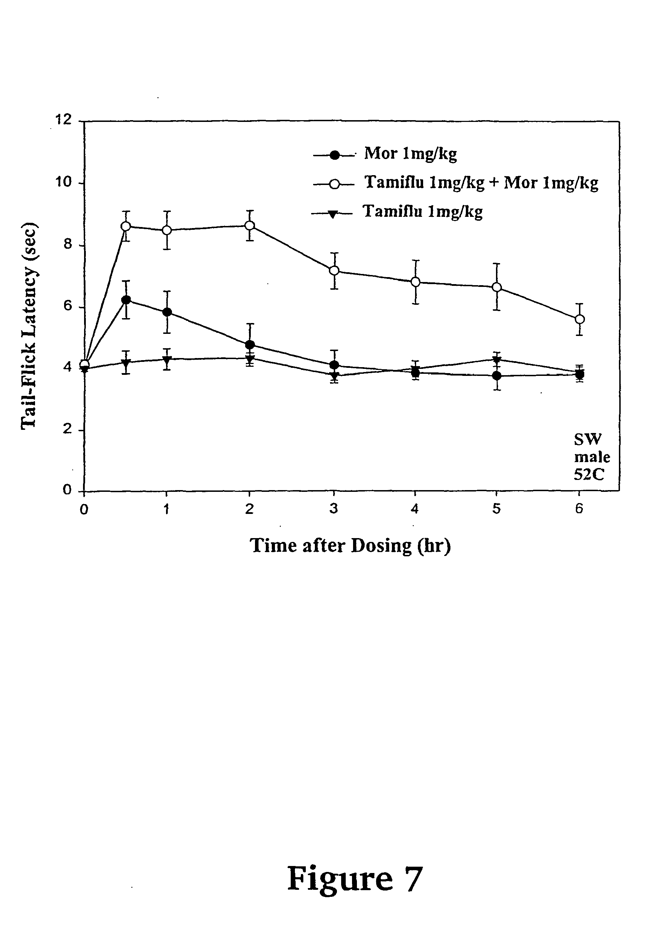

Figure 7 shows that cotreatment of male mice with oseltamivir (Tamiflu) markedly enhances the analgesic potency elicited by higher doses of morphine (Mor) (*cf*. ○ and ●).

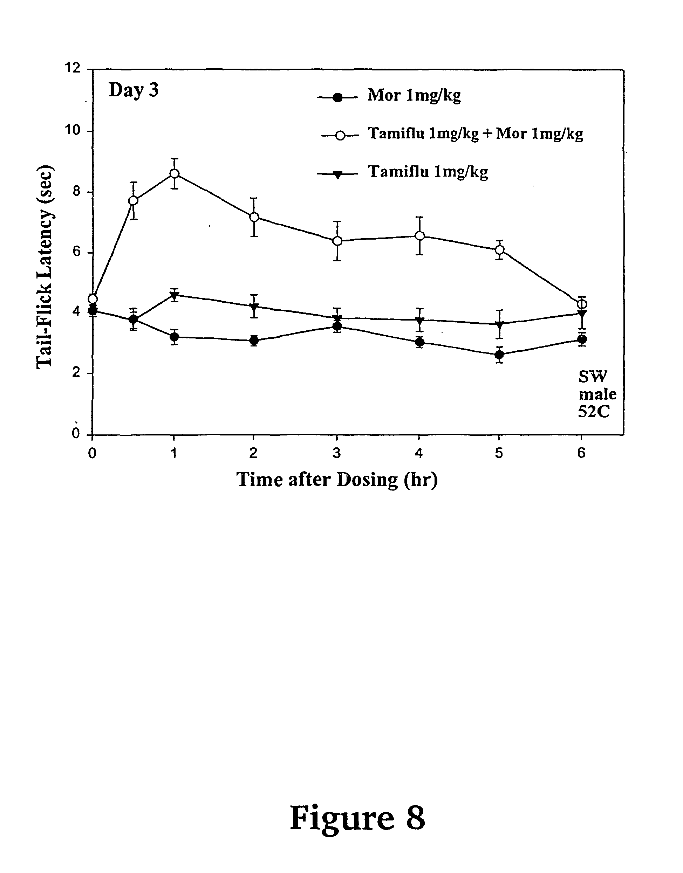

Figure 8 demonstrates that chronic cotreatment with oseltamivir (Tamiflu) plus morphine (Mor) for 3 days prevents development of opioid tolerance (*cf*. ○ and ●).

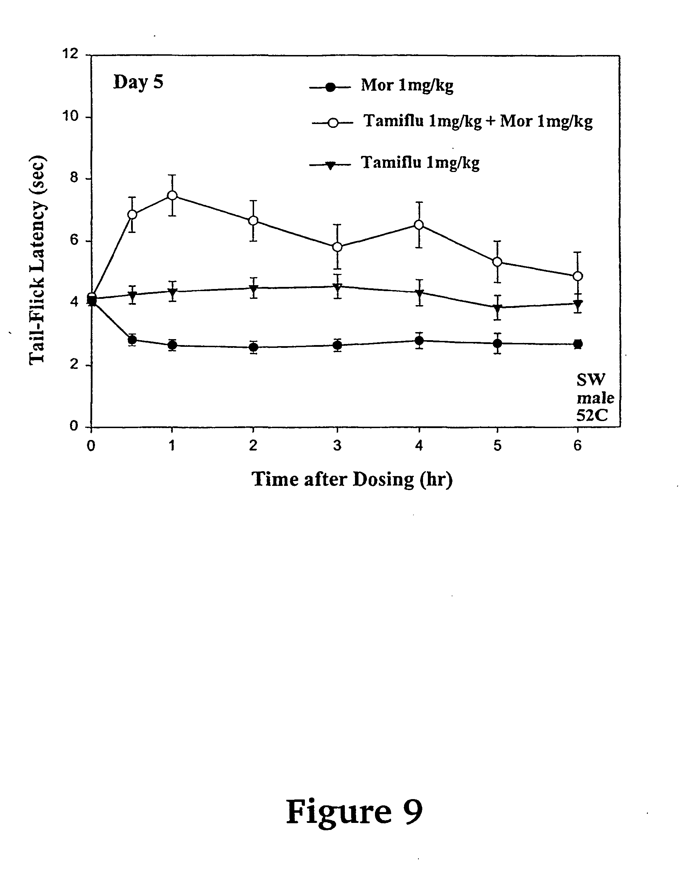

Figure 9 illustrates that mice treated with oseltamivir (Tamiflu) still show prominent morphine (Mor) analgesia after 5-days of cotreatment (○), whereas the morphine-only group shows marked hyperalgesia (●).

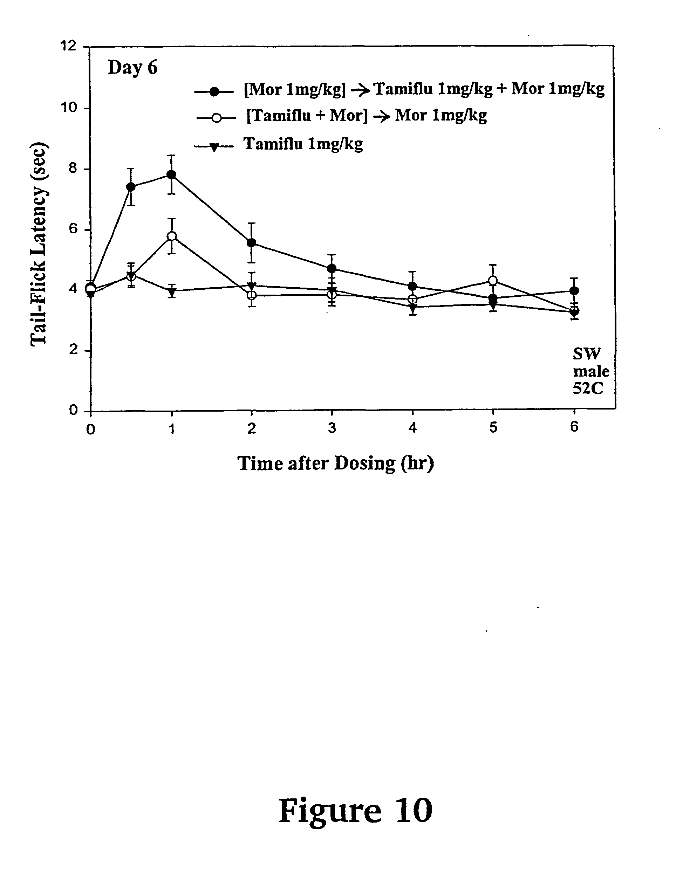

Figure 10 shows that addition of oseltamivir (Tamiflu) to the same group of chronic morphine-tolerant mice (shown in Fig. 9:●) for 1 day restores prominent analgesia (●), whereas morphine (Mor) tolerance develops within 1 day after withdrawal of oseltamivir (○).

### Detailed Description of the Invention

The present invention provides a method for increasing analgesic potency of a bimodally-acting (excitatory/inhibitory) opioid agonist in a subject. The subject is preferably a mammal (*e.g.*, humans, domestic animals, and commercial animals, including cows, dogs, mice, monkeys, pigs, and rats), and is most preferably a human.

As used herein, the term "opioid" refers to a natural or synthetic compound which binds to specific opioid receptors in the nervous system, and which has agonist (activation) or antagonist (inactivation) effects at these receptors. Examples of opioid compounds include, without limitation, opioid alkaloids (*e.g.*, the agonist morphine and the antagonist naloxone) and opioid peptides (*e.g.*, dynorphins, endorphins, and enkephalins). The term "opiate" refers to a drug derived from opium or related analogues.

As further used herein, "bimodally-acting opioid agonists" are opioid agonists that bind to, and activate, both inhibitory and excitatory opioid receptors on nociceptive neurons. "Nociceptive neurons", or nociceptors, are neurons which respond to stimuli that are damaging or potentially damaging to the skin (*e.g.*, intense pressure, high heat, and burning chemicals), and which thereby mediate pain. Analgesia, or relief from pain, results from activation by opioid agonists of inhibitory opioid receptors on neurons in the nociceptive (pain) pathways of the peripheral and central nervous systems. Adverse excitatory effects may result from sustained activation, by bimodally-acting opioid agonists, of excitatory opioid receptors on neurons in these nociceptive pathways. Examples of such adverse excitatory effects include, without limitation, anti-analgesia, hyperexcitability, hyperalgesia, physical dependence, psychological dependence, and tolerance, as well as constipation, nausea, respiratory depression, sedation, and vomiting.

The analgesic potency of a bimodally-acting opioid agonist may be increased by strengthening the agonist's activation of inhibitory opioid receptors in nociceptive neurons, which results in an augmented analgesic effect. Additionally, the analgesic potency of a bimodally-acting opioid agonist may be increased by reducing the agonist's activation of excitatory opioid receptors in nociceptive neurons. This attenuates anti-analgesia, hyperalgesia, hyperexcitability, physical dependence, psychological dependence, tolerance, and other adverse excitatory effects associated with administration of the bimodally-acting opioid agonist, thereby unmasking the agonist's potent opioid analgesia. Moreover, the analgesic potency of a bimodally-acting opioid agonist may be increased by strengthening the agonist's activation of inhibitory opioid receptors in nociceptive neurons while simultaneously reducing the agonist's activation of excitatory opioid receptors in nociceptive neurons.

In the method of the present invention, the analgesic potency of a bimodally-acting opioid agonist is increased in a subject by administering to the subject an analgesic or subanalgesic amount of the bimodally-acting opioid agonist, in combination with an agent that inhibits GM1-ganglioside in nociceptive neurons, in an amount effective to increase the analgesic potency of the bimodally-acting opioid agonist. It is also within the confines of the present invention that administration to a subject of an analgesic or subanalgesic amount of a bimodally-acting opioid agonist, in combination with an amount of an agent that inhibits GM1-ganglioside in nociceptive neurons, will increase the analgesic potency of a bimodally-acting opioid agonist while simultaneously attenuating adverse excitatory effects associated with administration of the bimodally-acting opioid agonist. Examples of such adverse excitatory effects include, without limitation, anti-analgesia, hyperalgesia, hyperexcitability, physical dependence, psychological dependence, and tolerance.

As disclosed herein, an agent that inhibits GM1-ganglioside in nociceptive neurons brings about a reduction in a bimodally-acting opioid agonist's activation of excitatory opioid receptors in nociceptive neurons. As a consequence, the agonist's activation of inhibitory opioid receptors is simultaneously unmasked. Thus, the reduction in activation of excitatory opioid receptors, combined with the unmasking of inhibitory opioid receptor effects, results in increased analgesic potency of a bimodally-acting opioid agonist when it is co-administered with an agent that inhibits GM1-ganglioside in nociceptive neurons. Accordingly, a much lower dose of the bimodally-acting opioid agonist may be administered to a subject than would otherwise be required in the absence of co-administration with an agent that inhibits GM1-ganglioside in nociceptive neurons. This has the added benefit of reducing or attenuating additional adverse effects associated with administration of opioid agonists (*e.g.*, constipation, nausea, respiratory depression, sedation, and vomiting) (3, 15).

The present invention can be used for administering to a subject an analgesic or subanalgesic amount of a bimodally-acting opioid agonist, in combination with an amount of an agent that inhibits GM1-ganglioside in nociceptive neurons. Bimodally-acting opioid agonists suitable for use in the present invention may be identified by measuring the opioid's effect on the action potential duration (APD) of dorsal root ganglion (DRG) neurons in tissue cultures. In this regard, bimodally-acting opioid agonists are compounds which elicit a prolongation of the APD of DRG neurons at picomolar (pM) to nanomolar (nM) concentrations (*e.g.*, excitatory effects), and a shortening of the APD of DRG neurons at µM concentrations (*e.g.*, inhibitory effects). Suitable bimodally-acting opioid agonists include, without limitation, buprenorphine, butorphanol, codeine, dynorphins, endorphins, enkephalins, fentanyl analogues, hydromorphone, levorphanol, meperidine, methadone, morphine, nalbuphine, oxycodone, oxymorphone, pentazocine, propoxyphene, tramadol, and other similarly-acting opioid alkaloids and opioid peptides. For the purposes of treating pain, morphine, codeine, and tramadol are preferred.

Gangliosides are a class of galactose-containing complex glycolipids (sphingolipids). They are found in highest concentration in the nervous system, particularly in gray matter, where they constitute 6% of the lipids. In gangliosides, an oligosaccharide chain containing at least one acidic sugar is attached to ceramide. The acidic sugar is *N*-acetylneuraminate or *N-*glycolylneuraminate - both of which are sialic acids. GM1 is a monosialoganglioside that is abundantly distributed on the external surface of neuronal cell membranes (23, 24, 25, 37, 62). It is formed by the addition of *N-*acetylgalactosamine and a galactose group. Cyclic AMP (cAMP) levels are known to stimulate neuronal levels of GM1-ganglioside, and the protein kinase A pathway is required for stimulating glycosyltransferase, the enzyme that makes GM1-ganglioside (11, 62).

As used herein, an agent that "inhibits GM1-ganglioside in nociceptive neurons" refers to an agent that reduces GM1-ganglioside activity in nociceptive neurons in a subject by disabling, disrupting, or inactivating the functions of GM1-ganglioside in nociceptive neurons in the subject, particularly the modulation or regulation of excitatory opioid receptors in nociceptive neurons, or by diminishing the levels or amount of GM1-ganglioside in nociceptive neurons in the subject. GM1-ganglioside in nociceptive neurons of a subject may be inhibited by targeting GM1-ganglioside directly. GM1-ganglioside in nociceptive neurons of a subject also may be inhibited indirectly, by targeting an enzyme or other endogenous molecule that regulates or modulates the functions or levels of GM1-ganglioside in nociceptive neurons in the subject. For example, an agent that inhibits GM1-ganglioside in nociceptive neurons may be an agent that is reactive with GM1-ganglioside. As used herein, "reactive" means the agent has affinity for, binds to, or is directed against GM1-ganglioside. Such an agent may block an allosteric GM1-binding site on excitatory opioid receptors. An agent that inhibits GM1-ganglioside in nociceptive neurons also may be an agent that regulates levels of GM1-ganglioside in nociceptive neurons in the subject.

Unless otherwise indicated, an "agent", as used herein, shall include a protein, polypeptide, peptide, nucleic acid (including DNA or RNA), antibody, Fab fragment, F(ab')₂ fragment, molecule, compound, antibiotic, drug, and any combinations thereof. A Fab fragment is a univalent antigen-binding fragment of an antibody, which is produced by papain digestion. A F(ab')₂ fragment is a divalent antigen-binding fragment of an antibody, which is produced by pepsin digestion. Examples of agents that inhibit GM1-ganglioside in nociceptive neurons include, without limitation, CTX-B, anti-GM1-ganglioside antibody, neuraminidase inhibitors (*e.g.*, Na₂SO₄, oseltamivir, and zanamivir), oligonucleotide antisense to CTX-B, agents that decrease or inhibit cAMP, and agents that decrease or inhibit glycosyltransferase - the enzyme that makes GM1-ganglioside. Preferably, the agent that inhibits GM1-ganglioside in nociceptive neurons is CTX-B or oseltamivir.

Cholera toxin consists of a noncovalently-assembled pentamer of a non-toxic B subunit (CTX-B) that is responsible for cell attachment, and a toxicogenic A subunit (CTX-A) (39). CTX-B binds with selective high affinity to GM1-ganglioside (K_{D}=10⁻¹⁰M) on the cell surface, and facilitates penetration of CTX-A into the cell membrane (27, 23, 43). As used herein, "CTX-B" refers to CTX-B and analogues and derivatives thereof, including, for example, a natural or synthetic functional variant of CTX-B which has CTX-B biological activity, as well as a fragment of CTX-B having CTX-B biological activity. As further used herein, the term "CTX-B biological activity" refers to the activity of a protein, peptide, or other molecule that demonstrates an ability to bind selectively to GM1-ganglioside, as described herein. CTX-B may be administered to a subject in doses ranging from 0.01-1 mg/kg per day.

CTX-B and its analogues and derivatives may be produced synthetically or recombinantly. For example, CTX-B and its analogues and derivatives may be produced and purified from a recombinant strain of *Vibrio cholerae* that lacks the CTX-A gene (45). CTX-B ("choleragenoid") and recombinant CTX-B are prepared in tablet form for oral administration, and may be obtained from List Biological Labs, Inc. (Campbell, CA). Furthermore, recombinant CTX-B (1 mg) is used in an oral cholera vaccine ("Dukoral") produced by SBL Vaccine (Stockholm, Sweden) (44). CTX-B in the form of a spray for nasal administration also is being developed for use as a vaccine (Maxim Pharmaceuticals, San Diego, CA).

In addition, CTX-B and CTX-B analogues may be isolated and purified from a culture of natural *Vibrio cholerae.* CTX-B protein may be isolated and purified from *Vibrio cholerae* using standard methods known in the art, including, without limitation, extraction (*e.g.*, with a detergent that solubilizes the protein), affinity purification on a column, chromatography (*e.g.*, FTLC and HPLC), immunoprecipitation (with an antibody to CTX-B), and precipitation (*e.g.*, with isopropanol and a reagent such as Trizol). Isolation and purification of CTX-B protein may be followed by electrophoresis (*e.g.*, on an SDS-polyacrylamide gel). CTX-B is preferably produced recombinantly, using a recombinant strain of *Vibrio cholerae* lacking the CTX-A gene.

Neuraminidase promotes release of influenza virus from infected cells, and facilitates virus spread within the respiratory tract. Several potent and specific inhibitors of this enzyme have been developed, and two (oseltamivir and zanamivir) have been approved for human use. Oseltamivir and zanamivir inhibit replication of both influenza A and B viruses. Early treatment with either drug reduces the severity and duration of influenza symptoms and associated complications (66).

Neuraminidase also regulates cellular levels of GM1-ganglioside. For example, administration of exogenous neuraminidase has been shown markedly to increase the concentrations of GM1-ganglioside in cell membranes of DRG cells and other neurons by enzymatic removal of neuraminic (sialic) acid from polysialylated ligands of the gangliotetraose series (11, 53, 59, 60, 64, 65, 69). This specific effect of neuraminidase on the enzymatic conversion of polysialylated gangliosides to GM1-ganglioside in neurons is quite distinct from the roles of neuraminidase in promoting release of influenza virus from infected cells and in facilitating virus spread within the respiratory tract.

In view of the foregoing, an agent that inhibits GM1-ganglioside in nociceptive neurons may be a neuraminidase inhibitor. Examples of neuraminidase inhibitors that may be useful in the present invention include, without limitation, Na₂SO₄, oseltamivir, zanamivir, and other similarly-acting neuraminidase inhibitors.

A recent study has demonstrated that inorganic sulfates (*e.g.*, Na₂SO₄ and MgSO₄) have potent inhibitory effects on mammalian neuraminidase activity in cultures of human macrophagic cells (70). Thus, these inorganic sulfates may be appropriate agents that inhibit GM1-ganglioside in nociceptive neurons, for use in the present invention. Na₂SO₄, for example, may be administered to a subject in need of treatment for pain in an amount on the order of 10 mg/kg per day.

Oseltamivir is an ethyl ester pro-drug that requires ester hydrolysis for conversion to the active form, oseltamivir carboxylate. The proposed mechanism of action of oseltamivir is *via* inhibition of influenza-virus neuraminidase, with the possibility of alteration of virus particle aggregation and release. Oseltamivir and its analogues and derivatives are available commercially. Oseltamivir is prepared in tablet form, for oral administration, under the trademark "Tamiflu", and may be obtained from Roche Laboratories (Nutley, NJ). Tamiflu is available as a capsule containing 75 mg of oseltamivir for oral use, in the form of oseltamivir phosphate. Tamiflu may be administered to a subject in a dose ranging from 0.1-1 mg/kg, once or twice a day.

Oseltamivir at doses that result in neuraminidase inhibition of influenza virus (66) also may be effective in decreasing GM1-ganglioside levels in nociceptive neurons. Such a decrease in levels of GM1-ganglioside would result in attenuation of the efficacy of GM1-regulated, Gₛ-coupled, excitatory opioid receptor-mediated hyperalgesic functions, thereby unmasking Gᵢ/Gₒ-coupled inhibitory opioid receptor-mediated analgesia and reducing development of tolerance and physical dependence.

In another embodiment of the present invention, the agent reactive with GM1-ganglioside is an antibody to GM1-ganglioside. The antibody of the present invention may be polyclonal or monoclonal, and may be produced by techniques well known to those skilled in the art. Polyclonal antibody, for example, may be produced by immunizing a mouse, rabbit, or rat with purified GM1-ganglioside. Monoclonal antibody then may be produced by removing the spleen from the immunized mouse, and fusing the spleen cells with myeloma cells to form a hybridoma which, when grown in culture, will produce a monoclonal antibody.

Other agents that inhibit GM1-ganglioside in nociceptive neurons may be identified using standard *in vitro* assays known in the art, including binding assays. For example, a candidate agent may be contacted with nociceptive neurons in cell culture, and the level of GM1-ganglioside expression in the cells may be determined using standard techniques, such as Western blot analysis. Similarly, a candidate agent may be contacted with nociceptive neurons in cell culture, and the level of GM1-ganglioside binding activity in the cells then may be determined using a CTX-B/peroxidase assay (62). Where the level of GM1-ganglioside expression or binding activity in nociceptive neurons is reduced in the presence of the candidate, it may be concluded that the candidate could be a useful agent that inhibits GM1-ganglioside in nociceptive neurons.

Once agents that inhibit GM1-ganglioside in nociceptive neurons have been identified, their suitability for use in the present invention may be ascertained by measuring their effects on the action potential duration (APD) of dorsal root ganglion (DRG) neurons in tissue cultures, as described in U.S. Patent No. 5,472,943. In particular, agents that inhibit GM1-ganglioside in nociceptive neurons of the present invention may be compounds which selectively block the prolongation of the APD of DRG neurons that is induced by low concentrations of morphine and other bimodally-acting opioid agonists (an excitatory opioid receptor-mediated effect) in DRG neurons, but not the shortening of the APD of DRG neurons (an inhibitory opioid receptor-mediated effect) that is elicited by bimodally-acting opioid receptor agonists. Alternatively, agents that inhibit GM1-ganglioside in nociceptive neurons of the present invention may be compounds which selectively block acute thermal hyperalgesia induced in mice by low concentrations (µg/kg) of morphine and other bimodally-acting opioid agonists (an excitatory opioid receptor-mediated effect), thereby unmasking potent opioid analgesia (15).

In the present invention, an analgesic or subanalgesic amount of a bimodally-acting opioid agonist is to be administered to a subject in combination with an agent that inhibits GM1-ganglioside in nociceptive neurons. As used herein, an "analgesic" amount is an amount of the bimodally-acting opioid agonist which causes analgesia (relief from pain) in a subject to whom the bimodally-acting opioid agonist is administered alone, and includes standard doses of the agonist which are typically administered to cause analgesia (*e.g.*, mg doses). A "subanalgesic" amount is an amount of the bimodally-acting opioid agonist which does not cause analgesia in a subject when administered alone to the subject, but which results in analgesia when used in combination with an agent that inhibits GM1-ganglioside in nociceptive neurons.

Inhibitory opioid receptor-mediated functions, which are activated by the bimodally-acting opioid agonist, are not attenuated by administration of the agent that inhibits GM1-ganglioside in nociceptive neurons. As a result, treatment with the agent that inhibits GM1-ganglioside in nociceptive neurons enhances the analgesic potency of the bimodally-acting opioid agonist, and reduces or attenuates adverse excitatory effects associated with administration of bimodally-acting opioid agonists (*e.g.,* anti-analgesia, hyperexcitability, hyperalgesia, physical dependence, psychological dependence, and tolerance, as well as constipation, nausea, respiratory depression, sedation, and vomiting).

When administered in combination with a bimodally-acting opioid agonist, an agent that inhibits GM1-ganglioside in nociceptive neurons may be administered in an amount that is effective to increase the analgesic potency of the bimodally-acting opioid agonist. As used herein, the term "effective to increase the analgesic potency of the bimodally-acting opioid agonist" is an amount that is effective to inactivate or attenuate excessive excitatory opioid receptor-mediated functions, such as anti-analgesia, hyperalgesia, hyperexcitability, physical dependence, tolerance, and other adverse effects, when it selectively blocks excitatory opioid receptor functions in nociceptive neurons. Accordingly, the agent that inhibits GM1-ganglioside in nociceptive neurons is administered at a dose which blocks the effects of the bimodally-acting opioid agonist on excitatory opioid receptors, without blocking the agonist's effects on inhibitory opioid receptors. This amount is readily determined by one skilled in the art (10, 14, 15, 52).

As described herein, the amount of GM1-ganglioside inhibitor for use in the present invention, relative to the amount of opioid agonist for use in the present invention, is not particularly crucial for achieving the augmented analgesic effects disclosed herein, because higher-than-necessary doses will not attenuate inhibitory analgesic effects. The optimum amounts of the bimodally-acting opioid agonist and the agent that inhibits GM1-ganglioside in nociceptive neurons to be administered will depend, of course, upon the particular factors of each case, including the agonist and agent used, the carrier chosen, the route of administration, and the physical and pharmacokinetic properties of the subject being treated. As described above, examples of doses of GM1-ganglioside inhibitors include, without limitation, 10 mg/kg of Na₂SO₄, 0.01-1 mg/kg of CTX-B, and 0.1-1 mg/kg of oseltamivir. Oseltamivir, may be administered to a subject in a dose ranging from 0.1-1 mg/kg, once or twice a day.

In the present invention, administration of a bimodally-acting opioid agonist "in combination with" an agent that inhibits GM1-ganglioside in nociceptive neurons refers to co-administration of the agonist and the agent. Co-administration may occur concurrently, sequentially, or alternately. Concurrent co-administration refers to administration of both the bimodally-acting opioid agonist and the agent that inhibits GM1-ganglioside in nociceptive neurons at essentially the same time. For concurrent co-administration, the courses of treatment with the bimodally-acting opioid agonist and with the agent that inhibits GM1-ganglioside in nociceptive neurons may be run simultaneously. For example, a single, combined formulation, containing both an amount of the bimodally-acting opioid agonist and an amount of the agent that inhibits GM1-ganglioside in nociceptive neurons, in physical association with one another, may be administered to the subject. The single, combined formulation may consist of an oral formulation, containing amounts of both the bimodally-acting opioid agonist and the agent that inhibits GM1-ganglioside in nociceptive neurons, which may be orally administered to the subject, or a liquid mixture, containing amounts of both the bimodally-acting opioid agonist and the agent that inhibits GM1-ganglioside in nociceptive neurons, which may be injected into the subject.

It is also within the confines of the present invention that an amount of a bimodally-acting opioid agonist and an amount of an agent that inhibits GM1-ganglioside in nociceptive neurons may be administered concurrently to a subject, in separate, individual formulations. For example, an amount of the agonist may be packaged in a vial or unit dose, and an amount of the agent may be packaged in a separate vial or unit dose, and the contents of the separate vials or unit doses then may be concurrently co-administered to the subject. Accordingly, the present invention is not limited to concurrent co-administration of the bimodally-acting opioid agonist and the agent that inhibits GM1-ganglioside in nociceptive neurons in physical association with one another.

In the present invention, a bimodally-acting opioid agonist and an agent that inhibits GM1-ganglioside in nociceptive neurons also may be co-administered to a subject in separate, individual formulations that are spaced out over a brief period of time (*e.g*., minutes or hours), so as to obtain the maximum efficacy of the combination. Administration of the agonist and the agent may range in duration from a brief, rapid administration, to a continuous perfusion. When spaced out over a brief period of time, co-administration of the bimodally-acting opioid agonist and the agent that inhibits GM1-ganglioside in nociceptive neurons may be sequential or alternate. For sequential co-administration, one of the compounds (*i*.*e*., either the agonist or the agent) is separately administered, followed by the other within a period of minutes or hours.

In accordance with the present invention, the bimodally-acting opioid agonist and the agent that inhibits GM1-ganglioside in nociceptive neurons (either in separate, individual formulations, or in a single, combined formulation) may be administered to a human or animal subject by known procedures, including, without limitation, oral administration, parenteral administration (*e*.*g*., epidural, epifascial, intracapsular, intracutaneous, intradermal, intramuscular, intraorbital, intraperitoneal (particularly in the case of localized regional therapies), intrasternal, intravascular, intravenous, parenchymatous, and subcutaneous administration), nasal administration, sublingual administration, transdermal administration, and administration by osmotic pump. Preferably, the GM1-ganglioside inhibitor of the present invention is administered nasally or orally.

For oral administration, formulations of the bimodally-acting opioid agonist and the agent that inhibits GM1-ganglioside in nociceptive neurons (whether individual or combined) may be presented in solid or liquid preparations, *e.g.,* capsules, tablets, powders, granules, dispersions, solutions, and suspensions. Such preparations are well known in the art, as are other oral dosage forms not listed here. The formulations may have conventional additives, such as lactose, mannitol, corn starch, or potato starch. The formulations also may be presented with binders, such as crystalline cellulose, cellulose derivatives, acacia, corn starch, or gelatins. Additionally, the formulations may be presented with disintegrators, such as corn starch, potato starch, or sodium carboxymethylcellulose. The formulations also may be presented with dibasic calcium phosphate anhydrous or sodium starch glycolate. Finally, the formulations may be presented with lubricants, such as talc or magnesium stearate.

For parenteral administration, formulations of the bimodally-acting opioid agonist and the agent that inhibits GM1-ganglioside in nociceptive neurons (whether individual or combined) may be combined with a sterile aqueous solution that is preferably isotonic with the blood of the subject. Such formulations may be prepared by dissolving a solid active ingredient in water containing physiologically-compatible substances, such as sodium chloride, glycine, and the like, and having a buffered pH compatible with physiological conditions, so as to produce an aqueous solution, then rendering said solution sterile. The formulations may be presented in unit or multi-dose containers, such as sealed ampules or vials. The formulations may be delivered by any mode of injection, including, without limitation, epidural, epifascial, intracapsular, intracutaneous, intradermal, intramuscular, intraorbital, intraperitoneal (particularly in the case of localized regional therapies), intrasternal, intravascular, intravenous, parenchymatous, or subcutaneous.

For nasal administration, aerosol, nasal-mist, or nasal-spray formulations of the bimodally-acting opioid agonist and the agent that inhibits GM1-ganglioside in nociceptive neurons (whether individual or combined) may be prepared in accordance with standard procedures known in the art for the preparation of nasal sprays. Moreover, CTX-B in the form of a spray for nasal administration may be obtained from Maxim Pharmaceuticals (San Diego, CA).

For transdermal administration, formulations of the bimodally-acting opioid agonist and the agent that inhibits GM1-ganglioside in nociceptive neurons (whether individual or combined) may be combined with skin penetration enhancers, such as propylene glycol, polyethylene glycol, isopropanol, ethanol, oleic acid, N-methylpyrrolidone, and the like, which increase the permeability of the skin to the agonist and the agent, and permit the agonist and the agent to penetrate through the skin and into the bloodstream. The composition of the enhancer and the agonist and/or agent also may be further combined with a polymeric substance, such as ethylcellulose, hydroxypropyl cellulose, ethylene/ vinylacetate, polyvinyl pyrrolidone, and the like, to provide the composition in gel form, which may be dissolved in a solvent, such as methylene chloride, evaporated to the desired viscosity, and then applied to backing material to provide a patch. The agonist and the agent may be administered transdermally, at or near the site on the subject where pain is localized. Alternatively, the agonist and the agent may be administered transdermally at a site other than the affected area, in order to achieve systemic administration.

Formulations of the bimodally-acting opioid agonist and the agent that inhibits GM1-ganglioside in nociceptive neurons (whether individual or combined) also may be released or delivered from an osmotic mini-pump or other time-release device. The release rate from an elementary osmotic mini-pump may be modulated with a microporous, fast-response gel disposed in the release orifice. An osmotic mini-pump would be useful for controlling release, or targeting delivery, of the agonist and the agent.

In accordance with the present invention, where the bimodally-acting opioid agonist or the agent that inhibits GM1-ganglioside in nociceptive neurons is a protein, the agonist or agent protein may be administered to a subject by introducing to the subject the agonist or agent protein itself, or by introducing to the subject a nucleic acid encoding the agonist or agent, in a manner permitting expression of the agonist or agent protein. The agonist and agent of the present invention may be introduced to the subject by known techniques used for the introduction of drugs, including, for example, injection and transfusion. Where pain is localized to a particular portion of the body of the subject, it may be desirable to introduce the agonist or agent directly to that area, by injection or by some other means (*e.g*., by introducing the agonist or agent into the blood or another body fluid).

For non-invasive introduction of agonists and agents, micro-encapsulated preparations, such as liposomes, also may be used. Liposomal vesicles may be prepared by various methods known in the art, and liposome compositions may be prepared using any one of a variety of conventional techniques for liposome preparation known to those skilled in the art. Examples of such methods and techniques include, without limitation, chelate dialysis, extrusion (with or without freeze-thaw), French press, homogenization, microemulsification, reverse phase evaporation, simple freeze-thaw, solvent dialysis, solvent infusion, solvent vaporization, sonication, and spontaneous formation. Preparation of the liposomes may be carried out in a solution, such as an aqueous saline solution, aqueous phosphate buffer solution, or sterile water. Liposome compositions also may be prepared by various processes involving shaking or vortexing. The agonist or agent may be incorporated into the layers of a liposome, such that its intracellular domain extends outside the liposome and its extracellular domain extends into the interior of the liposome. It is expected that liposomal delivery of a bimodally-acting opioid agonist or an agent that inhibits GM1-ganglioside in nociceptive neurons will facilitate passage of the agonist or agent through the blood-brain barrier (32).

Nucleic acid encoding the bimodally-acting opioid agonist or the agent that inhibits GM1-ganglioside in nociceptive neurons, as well as any antisense oligonucleotide or other nucleotide inhibitor of GM1-ganglioside, may be introduced to the subject using conventional procedures known in the art, including, without limitation, electroporation, DEAE Dextran transfection, calcium phosphate transfection, lipofection, monocationic liposome fusion, polycationic liposome fusion, protoplast fusion, creation of an in vivo electrical field, DNA-coated microprojectile bombardment, injection with recombinant replication-defective viruses, homologous recombination, in vivo gene therapy, ex vivo gene therapy, viral vectors, and naked DNA transfer, or any combination thereof. Recombinant viral vectors suitable for gene therapy include, but are not limited to, vectors derived from the genomes of viruses such as retrovirus, HSV, adenovirus, adeno-associated virus, Semiliki Forest virus, cytomegalovirus, and vaccinia virus.

It is also within the confines of the present invention that the formulations of the bimodally-acting opioid agonist and the agent that inhibits GM1-ganglioside in nociceptive neurons (either in separate, individual formulations, or in a single, combined formulation) may be further associated with a pharmaceutically-acceptable carrier, thereby comprising a pharmaceutical composition. Accordingly, the present invention provides a pharmaceutical composition, comprising a bimodally-acting opioid agonist, an agent that inhibits GM1-ganglioside in nociceptive neurons, and a pharmaceutically-acceptable carrier. The pharmaceutically-acceptable carrier must be "acceptable" in the sense of being compatible with the other ingredients of the composition, and not deleterious to the recipient thereof. Examples of acceptable pharmaceutical carriers include carboxymethyl cellulose, crystalline cellulose, glycerin, gum arabic, lactose, magnesium stearate, methyl cellulose, powders, saline, sodium alginate, sucrose, starch, talc, and water, among others. It is also within the confines of the present invention to provide a pharmaceutical composition comprising a bimodally-acting opioid agonist and a pharmaceutically-acceptable carrier, and a pharmaceutical composition comprising an agent that inhibits GM1-ganglioside in nociceptive neurons and a pharmaceutically-acceptable carrier.

The formulations of the pharmaceutical compositions of the present invention may be conveniently presented in unit dosage. The formulations also may be prepared by methods well-known in the pharmaceutical art. For example, the active compound may be brought into association with a carrier or diluent, as a suspension or solution. Optionally, one or more accessory ingredients (*e.g*., buffers, flavoring agents, surface active agents, and the like) also may be added. The choice of carrier will depend upon the route of administration. The pharmaceutical composition may be useful for administering the agonist and the agent of the present invention (*i*.*e*., the bimodally-acting opioid agonist, the agent that inhibits GM1-ganglioside in nociceptive neurons, and their analogues and derivatives, either in separate, individual formulations, or in a single, combined formulation) to a subject to treat pain.

In the composition of the present invention, the bimodally-acting opioid agonist may be any of those described above. Preferably, the agonist is morphine. Additionally, in the composition of the present invention, the agent that inhibits GM1-ganglioside in nociceptive neurons may be any of those described above. Preferably, the agent is CTX-B or oseltamivir. In the composition of the present invention, the bimodally-acting opioid agonist is provided in an analgesic amount, as defined above, or a subanalgesic amount, as defined above. The agent that inhibits GM1-ganglioside in nociceptive neurons is provided in an amount effective to increase the analgesic potency of the bimodally-acting opioid agonist in a subject, as defined above. These amounts may be readily determined by the skilled artisan, or in accordance with the methods described herein.

The present invention further can be used for treating pain in a subject in need of treatment thereof. Pain is a complex subjective sensation, reflecting real or potential tissue damage and the affective response thereto (3). Pain may be broadly classified as acute (lasting for hours or a few days) or chronic (persisting for more than one month), somatogenic or psychogenic. Somatogenic, or organic, pain may be explained in terms of physiologic mechanisms. Psychogenic pain occurs without an organic pathology sufficient to explain the degree of pain and disability, and is thought to be related mainly to psychologic issues (3).

Somatogenic pain may be nociceptive or neuropathic (3). Nociceptive pain results from ongoing activation of somatic or visceral pain-sensitive nerve fibers. When somatic nerves are affected, pain is typically felt as aching or pressure. Neuropathic pain results from dysfunction in the nervous system. It is believed to be sustained by aberrant somatosensory processes in the peripheral nervous system, the central nervous system, or both. Nociceptive pain may predominate in pain syndromes related to chronic joint or bone injury (*e.g.*, arthritis, cancer, hemophilia, and sickle cell disease). Common classes of pain include acute postoperative pain, cancer pain, headaches, neuropathic pain (*e.g.*, complex regional pain syndrome), and psychogenic pain syndromes (3). In the method of the present invention, the pain may be any of those described above, including acute pain and chronic pain, nociceptive pain and neuropathic pain. Preferably, the pain is nociceptive pain.

The present invention comprises the manufacture of a medicament for administering to a subject a bimodally-acting opioid agonist in combination with an agent that inhibits GM1-ganglioside in nociceptive neurons, in amounts effective to treat the pain in the subject. The subject may be any of those described above. Preferably, the subject is a human. A subject in need of treatment for pain may be a subject who is exhibiting or experiencing pain, or a subject who is about to be subjected to a pain-causing event.

In accordance with the method of the present invention, the bimodally-acting opioid agonist may be any of those described above. Preferably, the agonist is morphine. Additionally, the agent that inhibits GM1-ganglioside in nociceptive neurons may be any of those described above. Preferably, the agent is CTX-B or oseltamivir. The agonist and the agent of the present invention may be administered to a subject in need of treatment for pain by any of the methods, and in any of the formulations, described above. Preferably, the agonist and agent are administered nasally or orally. Moreover, the bimodally-acting opioid agonist and the agent that inhibits GM1-ganglioside in nociceptive neurons may be co-administered together in the same formulation, or they may be administered in separate formulations, as described above. If the agonist and the agent are co-administered in separate formulations, they may be co-administered by similar or different modes of administration, and they may be co-administered concurrently, sequentially, or alternately, as described above.

In the present invention, the agonist and the agent are to be administered to a subject in amounts effective to treat the pain in the subject. As used herein, the phrase "effective to treat the pain" means effective to ameliorate or minimize the clinical impairment or symptoms resulting from the pain (e.g., by diminishing any uncomfortable, unpleasant, or debilitating sensations experienced by the subject). The amounts of agonist and agent effective to treat pain in a subject in need of treatment thereof will vary depending on the particular factors of each case, including the type of pain, the location of the pain, the subject's weight, the severity of the subject's condition, the agonist and agent used, and the route of administration. These amounts can be readily determined by the skilled artisan.

In one embodiment of the present invention, the amount of the bimodally-acting opioid agonist is an analgesic or subanalgesic amount, as defined above, and the amount of the agent that inhibits GM1-ganglioside in nociceptive neurons is an amount effective to increase the analgesic potency of the bimodally-acting opioid agonist, as defined above. As described above, examples of doses of GM1-ganglioside inhibitors include, without limitation, 10 mg/kg of Na₂SO₄, 0.01-1 mg/kg of CTX-B, and 0.1-1 mg/kg of oseltamivir.

It is within the confines of the present invention that an agent that inhibits GM1-ganglioside, as described above, can be administered alone to chronic pain patients, in order to enhance the analgesic potency and reduce the tolerance/ dependence liability of endogenous opioid peptides, such as enkephalins, dynorphins, and endorphins, which are elevated in chronic pain patients (10). Accordingly, the present invention further provides a method for treating chronic pain in a subject in need of treatment thereof, comprising administering to the subject an agent that inhibits GM1-ganglioside in nociceptive neurons, in an amount effective to treat the chronic pain in the subject. The subject may be any of those described above. Preferably, the subject is a human. A subject in need of treatment for chronic pain may be a subject who is exhibiting or experiencing chronic pain. The agent that inhibits GM1-ganglioside in nociceptive neurons may be any of those described above. Preferably, the agent is CTX-B or oseltamivir.

The agent that inhibits GM1-ganglioside in nociceptive neurons may be administered to a subject by any of the methods, and in any of the formulations, described above. The agent is administered to a subject in an amount effective to treat the chronic pain in the subject. As used herein, the phrase "effective to treat the chronic pain" means effective to ameliorate or minimize the clinical impairment or symptoms resulting from the chronic pain (*e.g*., by diminishing any uncomfortable, unpleasant, or debilitating sensations experienced by the subject). The amount of agent effective to treat chronic pain in a subject in need of treatment thereof will vary depending upon the particular factors of each case, including the type of pain, the location of the pain, the subject's weight, the severity of the subject's condition, the agent used, and the method of administration. This amount can be readily determined by the skilled artisan.

In one embodiment of the present invention, the amount of the agent that inhibits GM1-ganglioside in nociceptive neurons is an amount effective to increase the analgesic potency of, and reduce the tolerance to or dependence on, endogenous opioid peptides, such as enkephalins, dynorphins, and endorphins, which are elevated in chronic pain patients. Thus, the agent that inhibits GM1-ganglioside may be administered in an amount that blocks the excitatory effects (*e.g.*, tolerance and physical dependence) of the endogenous bimodally-acting opioid agonist, without blocking the inhibitory effects (*e.g.*, analgesic effects) of the bimodally-acting opioid agonist. As described above, examples of doses of GM1-ganglioside inhibitors include, without limitation, 10 mg/kg of Na₂SO₄, 0.01-1 mg/kg of CTX-B, and 0.1-1 mg/kg of oseltamivir.

Excitatory opioid receptors in nociceptive neurons may become supersensitized by chronic exposure to endogenous or exogenous bimodally-acting (excitatory/inhibitory) opioid agonists, as has been shown to occur in somatic sensory dorsal root ganglion ("DRG") neurons. Neurons with supersensitized excitatory opioid receptor functions in nociceptive neurons, therefore, may become "physically dependent" on opioids, as occurs in somatic sensory and CNS neurons. Co-administration of 10⁻⁷ M CTX-B during chronic treatment of sensory neurons in culture with 10⁻⁶ M morphine or other bimodally-acting opioid agonists (> 1 week in culture) prevented development of the opioid excitatory supersensitivity, including naloxone-precipitated APD-prolongation, as well as the tolerance to opioid inhibitory effects that generally occurs after chronic opioid exposure. CTX-B binds selectively to allosteric GM1-ganglioside binding sites on excitatory opioid receptors, thereby blocking excitatory, but not inhibitory, opioid effects (50). Co-administration of CTX-B during chronic opioid treatment prevented the development of the plastic changes in neuronal sensitivity that are considered to be cellular manifestations related to opioid dependence and tolerance *in vivo* (51). In view of the foregoing, agents which inhibit GM1-ganglioside in nociceptive neurons may be useful for treating opioid-mediated addictions.

Accordingly, the present invention provides for treating adverse excitatory effects associated with administration of a bimodally-acting opioid agonist in a subject in need of treatment thereof. The bimodally-acting opioid agonist may be any of those described above. Preferably, the agonist is morphine. Examples of adverse excitatory effects include, without limitation, anti-analgesia, hyperexcitability, hyperalgesia, physical dependence, psychological dependence, and tolerance, as well as constipation, nausea, respiratory depression, sedation, and vomiting. As used herein, a "psychological dependence" is a psychological condition which manifests as an overpowering compulsion to continue taking opioids, and a "physical dependence" is a state of physiologic adaptation to a drug, which may increase in intensity with increased dosage and duration of use of opioids, and which may manifest in a withdrawal (abstinence) syndrome when the drug is discontinued or its effect is counteracted. As further used herein, "tolerance" refers to circumstances where the dosage of a bimodally-acting opioid agonist must be increased in order to obtain the initial effect.

The present invention can be used for administering to a subject an agent that inhibits GM1-ganglioside in nociceptive neurons, in an amount effective to treat the adverse excitatory effects in the subject. The subject may be any of those described above. Preferably, the subject is a human. The agent that inhibits GM1-ganglioside in nociceptive neurons may be any of those described above. Preferably, the agent is CTX-B or oseltamivir.

As used herein, the term "effective to treat the adverse excitatory effects" means effective to ameliorate or minimize the clinical impairment or symptoms resulting from the adverse excitatory effects (*e.g.*, by diminishing any tolerance to, or any psychological or physical dependence on, the opioid agonist). By attenuating tolerance to, and psychologic or physical dependence on, the opioid agonist, the agent that inhibits GM1-ganglioside in nociceptive neurons may produce added secondary benefits, including an increase in the analgesic potency of the bimodally-acting opioid agonist, and a decrease in side-effects associated with chronic opioid use or overdose and the withdrawal therefrom (*e.g.*, aching muscles, anorexia, anxiety, craving for the opioid, hot and cold flashes, increased respiratory rate, itching, lacrimation, muscle twitching, mydriasis, perspiration, piloerection, rhinorrhea, tremors, etc.).

The agent of the present invention may be administered to a subject by any of the methods, and in any of the formulations, described above. The amount of agent effective to treat tolerance to or an addiction to a bimodally-acting opioid agonist in a subject in need of treatment thereof will vary depending upon the particular factors of each case, including the type of tolerance or addiction, the extent of the tolerance or addiction, the subject's weight, the severity of the subject's condition, the agent used, and the method of administration. This amount can be readily determined by the skilled artisan. In one embodiment of the present invention, the amount of the agent that inhibits GM1-ganglioside in nociceptive neurons is an amount effective to increase the analgesic potency of the bimodally-acting opioid agonist, as defined above. Thus, the agent that inhibits GM1-ganglioside may be administered in an amount that blocks the excitatory effects (*e.g.*, tolerance and physical dependence) of the bimodally-acting opioid agonist, without blocking the inhibitory effects (*e.g.*, analgesic effects) of the bimodally-acting opioid agonist. This amount is readily determined by one skilled in the art. As described above, examples of doses of GM1-ganglioside inhibitors include, without limitation, 10 mg/kg of Na₂SO₄, 0.01-1 mg/kg of CTX-B, and 0.1-1 mg/kg of oseltamivir.

The present invention is described in the following Experimental Details section, which is set forth to aid in the understanding of the invention, and should not be construed to limit in any way the scope of the invention as defined in the claims which follow thereafter.

### Experimental Details

### 1. Introduction

Electrophysiologic studies of nociceptive types of mouse dorsal-root ganglion (DRG) neurons in culture have shown that specific µ, δ, and κ opioid agonists evoke naloxone-reversible prolongation of the action potential duration (APD), when applied to many of these neurons at low nanomolar (nM) concentrations (9, 48). In contrast, as shown in previous studies, higher micromolar (µM) levels of opioids shorten the APD (7, 57). This bimodal modulation of the APD in DRG neurons, by exposure to either low or high concentrations of opioid agonists, appears to be due to activation of excitatory or inhibitory opioid receptor functions.

Treatment with pertussis toxin (PTX), which uncouples inhibitory receptors linked to the regulatory G-proteins, Gᵢ and Gₒ (29, 36), blocks opioid-induced shortening of the APD in DRG neurons (48) and opioid depression of DRG-evoked postsynaptic dorsal-horn network responses in DRG-cord explants (16). Contrastingly, opioid-induced APD prolongation in DRG neurons is blocked by treatment with the A subunit of cholera toxin (CTX-A) (49), which interferes with ligand-activation of Gₛ-linked excitatory receptors.

CTX-A selectively catalyzes the ADP-ribosylation of Gₛ (28, 40), resulting in inhibition of an associated GTPase (6), increased adenylate cyclase activity, and decreased efficacy of ligand activation of Gₛ-coupled receptors (5, 6, 38, 41, 55). Brief treatment of DRG neurons with very low concentrations of purified CTX-A, or with whole toxin (1 pg/ml to 1 ng/ml), selectively blocks opioid-induced prolongation of the APD, thereby providing strong evidence for mediation of this excitatory modulatory effect by opioid receptors linked to a Gₛ/protein kinase A/cAMP second-messenger system (9, 17, 49).

Pretreatment of DRG neurons with CTX-B (1-10 ng/ml) also selectively blocks opioid-induced APD prolongation, but not opioid-induced APD shortening (49). In contrast to CTX-A blockade of opioid excitatory effects by ADP-ribosylation of Gₛ, CTX-B blockade appears to involve interference with GM1-ganglioside regulation of opioid excitatory receptor functions. This conclusion is based on the specificity of CTX-B in binding with selective high affinity (K_{D}= 10⁻¹⁰M) to GM1-ganglioside, which is abundantly distributed on the external surface of neuronal cell membranes (23, 24, 25, 37, 59). Treatment of DRG neurons with anti-GM1 antibodies also selectively blocks opioid-induced APD prolongation, as occurs with CTX-B (49).

Subsequent studies have shown that, following brief treatment of DRG neurons with low (10 nM) concentrations of GM1 (but not GM2, GM3, or other gangliosides or glycolipids), the threshold concentration of the opioid peptide dynorphin, which is required to prolong the APD in many DRG neurons, is markedly decreased from nM levels to fM-pM levels (49, 53). These electrophysiologic studies suggest that the increased sensitivity of GM1-treated DRG neurons to the excitatory effects of opioid agonists is due to GM1 binding to an allosteric regulatory site on opioid receptors (50), which appears to enhance the efficacy of excitatory, Gₛ-coupled opioid receptor functions (12).

The increased sensitivity of GM1-treated DRG neurons to the excitatory effects of opioid agonists is consonant with recent evidence that injection (i.p.) of low doses of GM1 (0.1 mg/kg) in mice rapidly attenuates morphine's analgesic effects (13). The present study provides more direct evidence that endogenous GM1-ganglioside plays a physiologic role in regulating excitatory opioid receptor functions in vivo, by demonstrating that remarkably low doses of CTX-B (10 ng/kg; s.c.) selectively block low-dose µ- and κ-opioid-induced hyperalgesic effects, thereby unmasking potent opioid analgesia. These results are comparable to the effects of cotreatment of mice with morphine and ultralow doses of NTX (10, 15, 52), which block low-dose, opioid-induced hyperalgesic effects, thereby unmasking potent opioid analgesia. Low-dose NTX selectively blocks excitatory opioid receptors at their recognition site, whereas CTX-B binds to, and interferes with, a putative allosteric GM1 regulatory site on excitatory opioid receptors (11, 12, 49). Furthermore, chronic cotreatment of mice with morphine and CTX-B also attenuates development of opioid tolerance and physical dependence, as previously shown to occur during cotreatment with low-dose NTX (10, 52).

### 2. Materials and Methods

### A. Antinociception and Hyperalgesia Assays in Mice

Swiss-Webster (SW) male and female mice (20-25 g; Charles River, NY) were housed separately in groups of five, maintained on a 12-hour light/dark cycle, and provided water and food *ad libitum* for 1-3 days prior to antinociception testing. Antinociceptive and hyperalgesic effects of opioids were measured on these mice using a hot-water-immersion tail-flick assay similar to those previously described (10, 13, 33, 52). Each mouse was permitted to enter into a tapered plastic cylinder with air holes. The size of the cylinder was slightly larger than the size of the animal's body, such that the tail could hang out freely from the cylinder. The cylinder provided a secluded environment into which the animal voluntarily entered with no application of force.

During the tail-flick assay, only the cylinder was handled, and there was not direct contact with the animal. One third of the tail, measured from the tip, was immersed into a water bath that was maintained at 52°C or 55°C (± 0.1°) with an electronic thermoregulator (Yellow Springs). The latency to a rapid tail-flick was recorded. Mice with control latencies > 8 sec were excluded from these tests, and a 10-second cutoff was used to minimize tissue damage. Six sequential control tests were made, each with a 10-minute interval. The latencies of the last 4 tests were averaged to provide a pre-drug value. Time-effect curves were plotted using tail-flick latencies as the ordinate (13).

### B. Animal Test Groups

Comparative tests were generally carried out on the same day, with two or more groups of 8 mice receiving a specific cotreatment, plus an appropriate control group of 8 mice treated with morphine (or other opioid test agonist) alone. Each animal test group was used for only one assay, except for the chronic drug treatment tests.

### C. Statistical Analyses

Differences between treatment groups were examined for statistical significance by means of ANOVA with Neuman-Keuls tests, or by means of a Students' t test (56).

### D. Materials

The following drugs were used: NTX, morphine, naloxone (now, and U-50,488H (Sigma); CTX-B ("choleragenoid") and recombinant CTX-B (List); and oseltamivir ("Tamiflu"; Roche Laboratories, Nutley, NJ). Stock solutions of CTX-B (commercially purified from whole CTX) were heated to 56°C for 20 min, to eliminate possible traces of CTX-A in the commercial product. This protocol was based upon the inventors' previous study that showed that the potent blocking effects of both CTX-A (tested up to 1 µg/ml) and whole CTX (tested up to 1 ng/ml) on opioid excitatory modulation of the APD were completely eliminated after this heat treatment (50). In contrast to the thermolabile, enzymatic properties of the A subunit, preparations of the B subunit of CTX showed relatively little loss in potency after heating to 56°C. These results demonstrate that pretreatment of DRG neurons with CTX-B can block opioid-induced APD prolongation, even under conditions which preclude effects mediated by the A subunit. Control tests were also carried out on some groups of mice that had been injected with recombinant CTX-B (rCTX-B), which had been produced and purified from a recombinant strain of *Vibrio cholerae* lacking the CTX-A gene (45).

### 3. Results

### A. Cotreatment of mice with CTX-B blocks acute, low-dose, morphine-induced thermal hyperalgesia, thereby unmasking potent opioid analgesia

Hot-water-immersion (52°C) nociceptive assays in normal, naive SW mice resulted in control tail-flick latencies of about 4 sec (15). Administration to these mice of very low doses of morphine (*ca*. 1 µg/kg; s.c.) resulted in rapid onset of decreases in tail-flick latencies (*ca*. 1-2 sec) which lasted for 4-5 h after drug injection (Fig. 1A:●). Cotreatment of other groups of mice with 1 µg/kg CTX-B (s.c.) blocked this low-dose, morphine-induced thermal hyperalgesia, and unmasked potent opioid analgesia lasting for > 6 h (Fig. 1A:▼) - effects similar to those produced by cotreatment with ultralow-dose NTX (Fig. 1A:○) (13). Cotreatment with a still lower dose of CTX-B (0.1 µg/kg) also was effective in blocking low-dose, morphine-induced hyperalgesia, and unmasking potent analgesia (Fig. 1B:▼).

In order to confirm that the inventors' heat treatment of regular CTX-B did, in fact, eliminate trace amounts of CTX-A, several groups of mice were tested with recombinant CTX-B. Cotreatment with the same or even lower doses of rCTX-B (10 ng/kg) also blocked low-dose, morphine-induced hyperalgesia, thereby unmasking potent opioid analgesia (Fig. 1C; *cf*. Fig. 1B).

### B. Cotreatment with CTX-B blocks acute, low-dose, κ-opioid-induced hyperalgesia, unmasking potent analgesia

Acute hyperalgesia elicited by the κ-opioid agonist, U-50,488H (10 ng/kg) (Fig. 2A:●), was blocked by CTX-B (0.1 µg/kg), thereby unmasking potent analgesia (Fig. 2A:∇). These effects were comparable to those which resulted from cotreatment with pg/kg doses of either NTX (Fig. 2A:○) or the specific κ-opioid antagonist, nor-binaltorphimine (Fig. 2A:▼). When the group of mice cotreated with U-50,488H plus CTX-B (Fig. 2A:∇) was retested 1 day later with the κ-opioid alone, the time-effect curve (Fig. 2B:○) showed that the blocking effect of CTX-B had disappeared, unmasking typical hyperalgesic responses (*cf*. Fig. 2A:●). Furthermore, when the group of mice showing low-dose, U-50,488H-induced hyperalgesia (Fig. 2A:●) was retested 1 day later, with U-50,488H plus a low dose of CTX-B (10 ng/kg), the hyperalgesia was blocked, unmasking prominent κ-opioid analgesia (Fig. 2B:●). Cotreatment of another group of mice with 10 ng/kg rCTX-B plus 10 ng/kg U-50,488H also blocked acute κ-opioid-induced hyperalgesia, unmasking potent analgesia, as occurred with regular CTX-B (Fig. 2C; *cf*. Fig. 2B).

### C. Acute cotreatment of mice with CTX-B markedly prolongs the analgesic effects of higher doses of morphine

A series of antinociceptive tail-flick assays of the effects of CTX-B cotreatment on morphine analgesia was also carried out using a slightly higher water-immersion temperature (55°C), as utilized in the inventors' previous studies with low-dose NTX cotreatment (52). In assays at 55°C, control tail-flick latencies were about 2 sec, making possible hyperalgesic effects more difficult to quantify (cf. Figs. 3 and 4 with Figs. 1 and 2). Cotreatment with 0.1 mg/kg CTX-B resulted in remarkable prolongation of the peak antinociceptive effect of 3 mg/kg morphine for > 5 h (Fig. 3:▼), whereas the effect of morphine alone sharply decreased after the first-hour peak (Fig. 3:●). CTX-B-induced enhancement of morphine's analgesic potency was comparable to, and perhaps even greater than, the enhancement that resulted from cotreatment with ultralow-dose NTX (Fig. 3:○) (10, 52). Similar results were obtained in several other assays of morphine ± CTX-B.

### D. Chronic cotreatment of mice with morphine plus CTX-B prevents the development of opioid tolerance

In another group of mice, acute cotreatment with CTX-B also markedly enhanced the magnitude, as well as the duration, of morphine's antinociceptive effects (Fig. 4A). After daily injections of 3 mg/kg morphine plus CTX-B for 5 days, the magnitude and duration of morphine's antinociceptive effects were still quite large (Fig. 4B:○). By contrast, the analgesic effects of morphine alone were sharply decreased by 5 days (Fig. 4B:●). The marked attenuation in morphine tolerance which resulted from cotreatment of morphine and CTX-B was similar to that which was observed with cotreatment of morphine and ultralow-dose NTX (52).

### E. Chronic cotreatment of mice with morphine plus CTX-B prevents development of NLX-precipitated withdrawal hyperalgesia

Injection of naive mice with low doses of NLX or NTX did not alter the baseline tail-flick latency in the inventors' antinociceptive assays (10, 52). In contrast, after chronic morphine treatment (*e.g*., 10 mg/kg for 5-6 days), withdrawal of morphine and injection of 10 µg/kg NLX evoked a prominent hyperalgesic response that lasted for more than 5 h. This response was comparable in magnitude and duration to that elicited by low-dose morphine in naïve mice (Figs. 1A, B:●). No significant NLX-precipitated withdrawal hyperalgesia occurred in mice that had been cotreated with CTX-B plus morphine. Interestingly, NLX-precipitated withdrawal hyperalgesia could be elicited in chronic morphine-treated mice well before the development of a marked degree of antinociceptive tolerance. This result is consonant with evidence of dependence without tolerance during chronic opioid treatment of DRG neurons in culture (51).

### F. Oral cotreatment of male and female mice with CTX-B blocks hyperalgesic effects of morphine injections, unmasking potent analgesia

CTX-B was added to the drinking-water bottles of groups of male, as well as female, mice for 1 day prior to antinociceptive assays using low-dose morphine (0.1 µg/kg; s.c.). Both male and female mice showed prominent, long-lasting antinociception after pretreatment with CTX-B, in sharp contrast to the marked hyperalgesia elicited by this low dose of morphine in control groups of mice (Figs. 5A and 5B). After a second day on oral CTX-B, the male and female mice were retested with a 10,000-fold higher dose of morphine (1 mg/kg; s.c.). Both the male and female groups treated with CTX-B showed much larger and longer-lasting analgesia than did the control groups that had received morphine alone (Figs. 5C and 5D). Although the magnitude of the antinociceptive effects elicited by morphine was consistently larger in the male groups, as compared with female groups, the efficacy of CTX-B cotreatment in enhancing morphine's analgesic potency was quite similar in all of the assays.

### G. Cotreatment of mice with oseltamivir and other neuraminidase inhibitors blocks excitatory opioid receptor functions by interference with GM1 regulation of these receptors

Administration of exogenous neuraminidase has been shown markedly to increase the concentrations of the monosialoganglioside GM1 in membranes of DRG and other neurons, by enzymatic removal of neuraminic (sialic) acid from polysialylated ligands of the gangliotetraose series in these neurons (11, 53, 59, 60, 64, 65, 69). This specific effect of neuraminidase on the enzymatic conversion of polysialylated gangliosides to GM1 in neurons is quite distinct from the roles of neuraminidase in promoting influenza virus release from infected cells and in facilitating virus spread within the respiratory tract. The antiviral effects of neuraminidase inhibitors may involve removal of sialic acid from cell surface glycoproteins, in contrast to removal of sialic acid from neuronal glycolipids.

Further studies, involving inhibitors of neuraminidase, have provided additional evidence that endogenous GM1-ganglioside plays a physiologic role in regulating excitatory opioid receptor-mediated functions *in vivo*. In particular, it was demonstrated that the neuraminidase inhibitor, oseltamivir (Tamiflu), selectively blocks morphine's hyperalgesic effects, thereby unmasking potent opioid analgesia (Figs. 6 and 7). It also was demonstrated that chronic cotreatment of mice with morphine plus oseltamivir attenuates development of opioid tolerance (Figs. 8 and 9). Interestingly, addition of oseltamivir to chronic morphine-tolerant mice restored prominent analgesia (Fig. 10). Chronic cotreatment with morphine plus oseltamivir also prevented development of NLX-precipitated withdrawal hyperalgesia. These results are comparable to those produced by cotreatment of mice with morphine plus ultralow-dose NTX or low-dose CTX-B, both of which block opioid-induced hyperalgesia, unmask potent opioid analgesia, and attenuate opioid tolerance and physical dependence (10, 52, 53, 63).

The present study in mice suggests that clinical administration of oseltamivir at doses that result in neuraminidase inhibition of influenza virus (66) concomitantly may be effective in decreasing GM1-ganglioside levels in nociceptive neurons. The latter result would attenuate the efficacy of GM1-regulated, Gₛ-coupled, excitatory opioid receptor-mediated hyperalgesic functions, thereby unmasking Gᵢ/Gₒ-coupled inhibitory opioid receptor-mediated analgesia and reducing development of tolerance and physical dependence. It is expected that cotreatment with oseltamivir plus morphine would result in similar effects in both sexes, as has been shown to occur in recent tests with CTX-B plus morphine in male and female mice.

Recently, it has been demonstrated that inorganic sulfates (*e.g.*, Na₂SO₄) have potent inhibitory effects on mammalian neuraminidase activity in cultures of human macrophagic cells (70). In view of the remarkable effectiveness of oseltamivir in enhancing morphine analgesia and attenuating tolerance/ dependence, as described above, the inventors carried out similar antinociception assays on mice cotreated with 3 µg/kg morphine plus 10 mg/kg Na₂SO₄. The results were surprisingly similar to those obtained with the much more complex neuraminidase inhibitor, oseltamivir. Chronic cotreatment of mice with 10 mg/kg of Na₂SO₄ plus 3 mg/kg of morphine enhanced analgesia, prevented development of tolerance during daily injections for 5 days, and prevented NLX-precipitated withdrawal hyperalgesia when tested on the sixth day. This unexpectedly simple procedure for enhancing morphine's analgesic potency and attenuating tolerance/dependence is counterintuitive, because several studies showing enhancement of morphine analgesia by cotreatment with MgSO₄ have been attributed to the antagonist effect of magnesium on excitatory NMDA-receptor functions, without recognition of the possible causal influence of sulfate (4, 21, 22, 42).

### 4. Discussion

The results of the present study provide strong evidence that GM1-binding to excitatory, Gₛ-coupled opioid receptors plays a physiologic role in regulating these receptors on neurons in nociceptive networks *in vivo*. The inventors previously showed that injection of low doses of exogenous GM1 (0.1-1 mg/kg; i.p.) in mice rapidly attenuates morphine's analgesic effects ("acute tolerance") (13), presumably by enhancing the efficacy of excitatory opioid receptor functions of nociceptive neurons. This interpretation is supported by the present evidence that injection of remarkably low doses of CTX-B (s.c.) rapidly blocks acute opioid-induced hyperalgesic effects, thereby unmasking potent opioid analgesia.

### A. Consonance of CTX-B blockade of opioid-induced hyperalgesia in mice and CTX-B blockade of excitatory opioid receptor-mediated functions in DRG neurons in culture

A possible role for GM1-ganglioside in regulating excitatory opioid receptor-mediated functions was initially proposed after it was observed that acute application of CTX-B to DRG neurons blocks excitatory, but not inhibitory, opioid receptor functions (50). Because CTX-B binds selectively to GM1-ganglioside on neuronal cell membranes, this result suggested that CTX-B might thereby interfere with a putative allosteric GM1 regulatory site on Gₛ-coupled excitatory opioid receptors (50).

Studies on DRG neurons also showed that CTX-blockade of opioid-induced excitatory, APD-prolonging effects in DRG neurons does not appear to be due to direct interference with ion-channel functions. Firstly, exposure to CTX-B alone did not result in significant alterations in the APD of DRG neurons. Secondly, exposure to forskolin resulted in characteristic prolongation of the APD in CTX-B treated neurons, demonstrating that CTX-B did not interfere with the responsiveness of cAMP-dependent ion channels, which mediate the excitatory effects of forskolin (30, 48, 58) and opioids (8, 9, 12, 48). Furthermore, in recent studies of non-opioid, GM1-deficient Chinese hamster ovary (CHO) cells transfected with cloned opioid receptors, acute application of GM1-ganglioside resulted in rapid (ca. 30 min) conversion of these receptors from an inhibitory, Gᵢ-coupled mode to an excitatory, Gₛ-coupled mode (11, 12, 61).

CTX-B blockade of acute, low-dose, opioid-induced hyperalgesia and unmasking of potent opioid analgesia in mice is remarkably consonant with the inventors' previous studies of CTX-B effects on DRG neurons in culture. Cotreatment of DRG neurons with 1-10 ng/ml CTX-B blocked low-dose (1-10 nM) µ-, δ-, and κ-opioid-induced excitatory, APD-prolonging ("hyperalgesic") effects in ∼ 15 min, unmasking potent opioid inhibitory APD-shortening ("analgesic") effects, which required 100- to 1,000-fold higher opioid concentrations when applied alone (50).

### B. Attenuation of opioid tolerance and physical dependence by chronic cotreatment with CTX-B

Attenuation of tolerance in chronic opioid-exposed mice resulted from cotreatment with CTX-B. This effect is consonant with the inventors' previous study on DRG neurons in culture, which showed that chronic cotreatment with nM concentrations of CTX-B attenuated development of tolerance to the inhibitory, APD-shortening effects of µM concentrations of the stable δ/µ opioid peptide, D-ala²-D-leu⁵-enkephalin (DADLE) (51).

Attenuation of the development of tolerance in chronic opioid-treated DRG neurons in culture, and in mice *in vivo,* by cotreatment with CTX-B or low-dose NTX (10, 52) can be attributed to the selective blockade of excitatory, Gₛ-coupled opioid receptor functions (which would otherwise be upregulated and supersensitized during chronic exposure to a bimodally-acting opioid agonist), thereby unmasking inhibitory opioid receptor functions (10, 12, 51, 74). It should be noted, however, that, in both the present in vivo study (Figs. 4A and 4B) and the inventors' earlier *in vitro* study (51), a small but significant degree of tolerance was observed in the CTX-B-treated test groups. This observed tolerance probably was due to homologous desensitization of inhibitory opioid receptor functions (71, 72, 74).

Additionally, prevention of NLX-precipitated withdrawal hyperalgesia in chronic morphine-treated mice, by cotreatment with CTX-B, is remarkably consonant with the inventors' study of chronic opioid-treated DRG neurons in culture, in which NLX-precipitated, excitatory, APD-prolonging effects in opioid-supersensitized neurons (10, 74) were prevented by chronic cotreatment with CTX-B (51). The present demonstration of NLX-precipitated withdrawal hyperalgesia in chronic morphine-treated mice presents a valuable new assay that may provide a measure of spinally-mediated physical dependence, thereby extending previous studies of NLX-precipitated withdrawal-jumping which may involve brainstem, as well as spinal, mechanisms (12). Interestingly, the low dose of NLX (10 µg/kg) used to evoke hyperalgesia in chronic morphine-treated mice was comparable to doses used clinically to elicit severe autonomic withdrawal symptoms in opiate-dependent humans (73).

### C. CTX-B cotreatment blocks κ- as well as µ-opioid-induced hyperalgesia in mice

The present study demonstrates that the acute hyperalgesic effects of low doses of the specific κ-opioid agonist, U-50,488H, as well as the µ agonist, morphine, are blocked by CTX-B, such that potent opioid analgesia is unmasked. Hyperalgesia induced in mice by 10 ng/kg U-50,488H is an effect that is consonant with a previous report (1) that indicated that a low dose of the κ-opioid peptide, dynorphin (0.5 µg/kg; s.c.), resulted in thermal hyperalgesia in mice, as measured by a significant decrease in tail-flick latency (tested at 90 min post-injection). In contrast, a 100-fold higher dose (50 µg/kg) elicited analgesia (1). The present study extends the previous report (1) by showing that low-dose, κ-opioid-induced hyperalgesia can be selectively blocked by ultralow doses of the specific κ-opioid antagonist, nor-binaltorphimine, and by NTX and CTX-B. The inventors' results also are in agreement with evidence that the hyperalgesia observed within 1 h after i.v. injection of a low, 5-mg dose of the κ-opioid agonist, nalbuphine, in male dental-pain patients is blocked by cotreatment with 0.5 mg naloxone, unmasking prominent analgesia (26).

### D. Efficacy and reversibility of CTX-B in blocking opioid-induced hyperalgesia

It is surprising that subcutaneous injection of doses of CTX-B as low as 10-100 ng can reach sufficient numbers of putative allosteric GM1-binding sites on excitatory opioid receptors on nociceptive neurons, so as to block low-dose, opioid-induced hyperalgesia in <30 min after administration. Perhaps some of the CTX-B molecules can enter the spinal cord via dorsal roots, and thereby contact opioid receptors on presynaptic terminals of nociceptive DRG neurons, as the inventors have suggested may account for the similarly surprising enhancement of excitatory opioid effects by intraperitoneal injection of low doses of exogenous GM1 in mice (13).

CTX-B association with GM1-containing membranes appears to result in a localized disturbance of membrane phospholipid packing (35). Initial disruption of membrane packing by CTX-B has been proposed to result from the rigid placement of five GM1 molecules within the membrane, following association with CTX-B (43). This arrangement could create a geometry in which the area available for occupation by phospholipids is reduced by 25% or more between the fixed positions of the five GM1 molecules bound by the CTX-B pentamer (43, 31). Such physicochemical interactions may underlie the efficacy of CTX-B in binding to GM1 sites on excitatory opioid receptors, thereby blocking opioid-induced hyperalgesia.

Interestingly, CTX-B is currently utilized clinically in oral and nasal mucosal vaccines (32, 44), and its immunopotentiating capacity as a carrier molecule is considered to be related to its ability to bind GM1 on the cell membranes of immunocytes (20, 34, 54). In view of the clinical studies with oral CTX-B, the inventors carried out preliminary tests in mice to determine the effects of orally-administered CTX-B on opioid analgesia. Injection of low-dose morphine (s.c.) in mice pretreated with oral CTX-B (via their drinking water) resulted in analgesia, rather than hyperalgesia (Fig. 5), comparable to the effects observed with s.c. injection of CTX-B (Fig. 1). Notably, the enhancing effects of oral CTX-B on morphine analgesia were quite similar in both male and female mice (Fig. 5).

The inventors' antinociceptive assays in mice cotreated with morphine plus CTX-B demonstrate that CTX-B blockade of opioid hyperalgesia and enhancement of analgesia is effective for >6 h after drug injection (Fig. 3). However, the blocking effect of 10 ng/kg CTX-B on low-dose, opioid-induced hyperalgesia was no longer present when the mice were retested 24 h later with opioid alone (Fig. 2B). Nevertheless, the results of chronic, daily cotreatment with 0.1 mg/kg CTX-B and morphine indicate that CTX-B blockade of opioid hyperalgesia and tolerance can be maintained quite effectively, even after 5 days (Fig. 4B).

### E. Cotreatment of mice with whole CTX blocks excitatory opioid receptor functions by CTX-A-mediated interference with Gₛ coupling, whereas CTX-B interferes with GM1 regulation of these receptors

Intracerebroventricular (i.c.v.) injection of whole CTX in mice has been reported to markedly enhance the analgesic potency of morphine, presumably by "impairing the function of Gₛ transducer proteins" (46). Furthermore, it has been shown that intrathecal injection of whole CTX in mice inhibits the "antianalgesic action" of dynorphin, which appeared to be "mediated by activation of [excitatory] Gₛ-coupled opioid receptors" (2). These interpretations are supported by the following evidence: (1) i.c.v. injection of mice with antibodies directed against G_{sα} also enhance morphine antinociception (47); and (2) downregulation of the G_{sα} protein by intrathecal injection of antisense oligonucleotides in mice results in blockade of low-dose, morphine-induced hyperalgesia (19) and tolerance during chronic morphine treatment (18).

The studies with whole, CTX in mice are consistent with selective blockade of excitatory opioid receptor functions by low-dose, CTX-A-mediated interference with Gₛ coupling of these receptors, as previously demonstrated with both whole-CTX-treated and CTX-A-treated DRG neurons in culture (49). In contrast, the present study shows that cotreatment with the non-toxic CTX-B subunit can result in remarkably similar blockade of excitatory opioid receptor functions by interference with GM1 regulation of these Gₛ-coupled receptors.

### References

1. Apfel et al., Kappa opioid receptors participate in nerve growth factor-induced hyperalgesia. Neurosci., 68:1199-1206, 1995.
2. Arts et al., Inhibition of the antianalgesic action of dynorphin A in mice by cholera toxin. Pharmacol. Biochem. & Behavior, 46:623-28, 1993.
3. Beers and Berkow (eds.), The Merck Manual of Diagnosis and Therapy, 17th ed. (Whitehouse Station, NJ: Merck Research Laboratories, 1999) 1363, 1369-1375, 1585.
4. Kroin et al., Magnesium sulfate potentiates morphine antinociception at the spinal level. Anesth. Analg., 90:913-17, 2000.
5. Casey and Gilman, G protein involvement in receptor-effector coupling. J. Biol. Chem., 263:2577-80, 1988.
6. Cassel and Selinger, Mechanism of adenylate cyclase activation by cholera toxin: inhibition of GTP hydrolysis at the regulatory site. Proc. Natl. Acad. Sci. USA, 74:3307-11, 1977.
7. Chalazonitis and Crain, Maturation of opioid sensitivity of fetal mouse dorsal-root ganglion neuron perikarya in organotypic cultures: regulation by spinal cord. Neurosci., 17:1181-98, 1986.
8. Chen et al., Inhibitor of cyclic AMP-dependent protein kinase blocks opioid-induced prolongation of the action potential of mouse sensory ganglion neurons in dissociated cell culture. Brain Res, 462:372-77, 1988.
9. Crain and Shen, Opioids can evoke direct receptor-mediated excitatory effects on sensory neurons. Trends in Pharmacol. Sci., 11:77-81, 1990.
10. Crain and Shen, Ultra-low concentrations of naloxone selectively antagonize excitatory effects of morphine on sensory neurons, thereby increasing its antinociceptive potency and attenuating tolerance/dependence during chronic cotreatment. Proc. Natl. Acad Sci. USA, 92:10,540-44, 1995.
11. Crain and Shen, GM1 ganglioside-induced modulation of opioid receptor-mediated functions. Ann. N.Y. Acad. Sci., 845:106-25, 1998.
12. Crain and Shen, Modulation of opioid analgesia, tolerance and dependence by Gs-coupled, GM1 ganglioside-regulated opioid receptor functions. Trends in PharmacoL Sci., 19:358-65, 1998.
13. Crain and Shen, Enhanced analgesic potency and reduced tolerance of morphine in 129/SvEv mice: evidence for a deficiency in GM1 ganglioside-regulated excitatory opioid receptor functions. Brain Res., 856:227-35, 2000.
14. Crain and Shen, Antagonists of excitatory opioid receptor functions enhance morphine's analgesic potency and attenuate opioid tolerance/ dependence liability. Pain, 84:121-31, 2000.
15. Crain and Shen, Acute thermal hyperalgesia elicited by low-dose morphine in normal mice is blocked by ultra-low naltrexone, unmasking potent opioid analgesia. Brain Res., 888:75-82, 2001.
16. Crain et al., Pertussis toxin blocks depressant effect of opioid, monoaminergic and muscarinic agonists on dorsal-horn network responses in spinal cord-ganglion cultures. Brain Res., 400:185-90, 1987.
17. Cruciani et al., Direct coupling of opioid receptors to both Gs and Gi proteins in F11 neuroblastoma X sensory neuron hybrid cells. Proc. Natl. Acad. Sci. USA, 90:3019-23, 1993.
18. Cruciani et al., Antisense oligonucleotides to Gsα prevents the decline of morphine analgesia over time in mice. Soc. Neurosci. Abstr., 25:1478, 1999.
19. Cruciani and Pasternak, Blockade of hyperalgesia to morphine by antisense oligodeoxynucleotides to Gsα. Internat. Narcot. Res. Conf. Abstr., 59, 2000.
20. Dertzbaugh and Elson, Reduction in oral immunogenicity of cholera toxin B subunit by N-terminal peptide addition. Infect. Immun., 61:384-90, 1993.
21. Koinig et al., Magnesium sulfate reduces intra- and postoperative analgesic requirements. Anesth. Analg., 87:206-10, 1998.
22. McCarthy et al., Antinociceptive potentiation and attenuation of tolerance by intrathecal co-infusion of magnesium sulfate and morphine in rats. Anesth. Analg., 86:830-36, 1998.
23. Fishman, P.H., Role of membrane gangliosides in the binding and action of bacterial toxins. J. Membr. Biol., 69:85-97, 1982.
24. Fishman, P.H., Recent advances in identifying the functions of gangliosides. Chem. Phys. Lipids., 42:137-51, 1986.
25. Fishman, P.H., Gangliosides and cell surface receptors and transducers of biological signals. In New Trends in Ganglioside Research: Neurochemical and Neuroregenerative Aspects, R.W. Ledeen, E.L. Hogan, G. Tettamenti, A.J. Yates, and R.K. Yu (eds.) (Padova: Liviana, 1988) 183-201.
26. Gear et al., Action of naloxone on gender-dependent analgesic and antianalgesic effects of nalbuphine in humans. J. Pain, 1:122-27, 2000.
27. Gill, D.M., Mechanism of action of cholera toxin. Adv. Cycl. Nucl. Res., 8:85-118, 1977.
28. Gill and Meren, ADP-ribosylation of membrane proteins catalyzed by cholera toxin: basis of the activation of adenylate cyclase. Proc. Natl. Acad. Sci. USA, 75:3050-54, 1978.
29. Gilman, A.G., G protein and dual control of adenylate cyclase. Cell, 36:577-79, 1984.
30. Grega and Macdonald, Activators of adenylate cyclase and cyclic AMP prolong calcium-dependent action potential of mouse sensory neurons in culture by reducing a voltage-dependent potassium conductance. J. Neurosci., 7:700-07, 1987.
31. Hardy et al., Coordinated assembly of multisubunit proteins: oligomerization of bacterial enterotoxins in vivo and in vitro. Proc. Natl. Acad. Sci. U.S.A., 85:7109-13, 1988.
32. Harokopakis et al., Effectiveness of liposomes possessing surface-linked recombinant B subunit of cholera toxin as an oral antigen delivery system. Infect. and Immun., 66:4299-304, 1998.
33. Heyman et al., Opioid receptor involvement in supraspinal and spinal antinociception in mice. Brain Res., 420:100-08, 1987.
34. Holmgren et al., Cholera toxin and cholera B subunit as oral mucosal adjuvant and antigen vector systems. Vaccine, 11:1179-84, 1993.
35. Krasilnikov et al., The ionic channels formed by cholera toxin in planar bilayer lipid membranes are entirely attributable to its B-subunit. Biochim. Biophys. Acta, 1067:166-70, 1991.
36. Kurose et al., Specific uncoupling by islet-activating protein, pertussis toxin, of negative signal transduction via alpha-adrenergic, cholinergic, and opiate receptors in neuroblastoma x glioma hybrid cells. J. Biol. Chem., 258:4870-75, 1983.
37. Ledeen, R.W., Biosynthesis, metabolism, and biological effects of gangliosides. In Neurobiology of Glycoconjugates, R.V. Margolis and R.K. Margolis (eds.) (New York: Plenum, 1989) 43-83.
38. Lux and Schulz, Effect of cholera toxin and pertussis toxin on opioid tolerance and dependence in the guinea-pig myenteric plexus. J. Pharmacol. Exp. Ther., 237:995-1000, 1986.
39. Mekalanos et al., Cholera toxin gene: nucleotide sequence, deletion analysis and vaccine development. Nature, 306:551-57, 1983.
40. Moss and Vaughan, Mechanism of action of choleragen and E. coli heat-labile enterotoxin: activation of adenylate cyclase by ADP-ribosylation. Mol. Cell. Biochem., 37:75-90, 1981.
41. Moss and Vaughan, Cholera toxin and E. coli enterotoxins and their mechanisms of action. In Handbook of Natural Toxins, vol. 4, M.C. Hardegree and A.T. Tu (eds.) (New York: Dekker, 1988) 39-87.
42. Tramer et al., Role of magnesium sulfate in postoperative analgesia. Anesthesiol., 84:340-47, 1996.
43. Ribi et al., Three-dimensional structure of cholera toxin penetrating a lipid membrane. Science, 239:1272-76, 1988.
44. Rudin et al., Differential kinetics and distribution of antibodies in serum and nasal and vaginal secretions after nasal and oral vaccination of humans. Infect. Immun., 66:3390-96, 1998.
45. Sanchez and Holmgren, Recombinant system for overexpression of cholera toxin B subunit in Vibrio cholerae as a basis for vaccine development. Proc. Natl. Acad. Sci. U.S.A., 86:481-85, 1989.
46. Sanchez-Blazquez and Garzon, Cholera toxin and pertussis toxin on opioid- and α2-mediated supraspinal analgesia in mice. Life Sci., 48:1721-27, 1991.
47. Sanchez-Blazquez and Garzon, Intracerebroventricular injection of antibodies directed against Gs enhances the supraspinal antinociception induced by morphine, β-endorphin and clonidine in mice. Life Sci., 51:PL237-PL242, 1992.
48. Shen and Crain, Dual opioid modulation of the action potential duration of mouse dorsal root ganglion neurons in culture. Brain Res., 491:227-42, 1989.
49. Shen and Crain, Cholera toxin-A subunit blocks opioid excitatory effects on sensory neuron action potentials indicating mediation by Gs-linked opioid receptors. Brain Res., 525:225-31, 1990.
50. Shen and Crain, Cholera toxin-B subunit blocks opioid excitatory effects on sensory neuron action potentials indicating that GM1 ganglioside may regulate Gs-linked opioid receptor functions. Brain Res., 531:1-7, 1990.
51. Shen and Crain, Chronic selective activation of excitatory opioid receptor functions in sensory neurons results in opioid "dependence" without tolerance. Brain Res., 597:74-83, 1992.
52. Shen and Crain, Ultra-low doses of naltrexone or etorphine increase morphine's antinociceptive potency and attenuate tolerance/dependence in mice. Brain Res., 757:176-90, 1997.
53. Shen et al., Brief treatment of sensory ganglion neurons with GM1 ganglioside enhances the efficacy of opioid excitatory effects on the action potential. Brain Res., 550:130-38, 1991.
54. Staats et al., Mucosal immunity to infection with implications for vaccine development. Curr. Opin. Immunol., 6:572-83, 1994.
55. Stadel and Lefkowitz, Differential effects of cholera toxin on guanine nucleotide regulation of beta-adrenergic agonist high affinity binding and adenylate cyclase activation in frog erythrocyte membranes. J. Cycl. Nucl: Res., 7:363-74, 1981.
56. Tallarida and Murray, Manual of Pharmacologic Calculations with Computer Programs, 2nd ed. (New York: Springer-Verlag, 1986).
57. Werz and Macdonald, Dynorphin and neoendorphin peptides decrease dorsal root ganglion neuron calcium-dependent action potential duration. J. Pharmacol. Exp. Therap., 234:49-56, 1985.
58. Womble and Wickelgren, Inhibition of a cAMP-dependent Ca-activated K conductance by forskolin prolongs calcium action potential duration in lamprey sensory neurons. Brain Res., 518:135-42, 1990.
59. Wu and Ledeen, Stimulation of neurite outgrowth in neuroblastoma cells by neuraminidase: putative role of GM1 ganglioside in differentiation. J. Neurochem., 56:95-104, 1991.
60. Wu et al., GM1 ganglioside modulates prostaglandin E1 stimulated adenylyl cyclase in neuro-2A cells. Glycoconjugate J., 13:235-39, 1996.
61. Wu et al., Interaction of the δ-opioid receptor with GM1 ganglioside: conversion from inhibitory to excitatory mode. Molec. Brain Res., 44:341-46, 1997.
62. Wu et al., The role of GM1 ganglioside in regulating excitatory opioid effects. Ann. N.Y. Acad. Sci., 845:126-38, 1998.
63. Abul-Husn et al., Paradoxical augmentation of morphine antinociception and inhibition of tolerance by ultra-low dose of the opioid antagonist naltrexone. Soc. Neurosci. Abstr., 26:1665, 2000.
64. Wieraszko and Seifert, The role of monosialoganglioside GM1 in the synaptic plasticity: in vitro study on rat hippocampal slices. Brain Res., 345:159-64, 1985.
65. Doherty and Walsh, Ganglioside GM1 antibodies and B-cholera toxin bind specifically to embryonic chick dorsal root ganglion neurons but do not modulate neurite regeneration. J. Neurochem., 48:1237-44, 1987.
66. Gubareva et al., Influenza virus neuraminidase inhibitors, Lancet, 355:827-35, 2000.
67. Mao et al., Thermal hyperalgesia in association with the development of morphine tolerance in rats: roles of excitatory amino acid receptors and protein kinase C. J. Neurosci., 14(4):2301-12, 1994.
68. Mayer et al., The development of morphine tolerance and dependence is associated with translocation of protein kinase C. Pain, 61(3):365-74, 1995.
69. Kopitz et al., Effects of cell surface ganglioside sialidase inhibition on growth control and differentiation of human neuroblastoma cells. Eur. J. Cell Biol., 73:1-9, 1997.
70. Greffard et al., Inhibition of acid sialidase by inorganic sulfate. Biochim. Biophysica. Acta., 1334:140-48, 1997.
71. Bohn et al., Enhanced morphine analgesia in mice lacking β-arrestin 2. Science, 286:1495-98, 1999.
72. Bohn et al., µ-opioid receptor desensitization by β-arrestin-2 determines morphine tolerance but not dependence. Nature, 408:720-23, 2000.
73. Wikler, Opioid Dependence (New York: Plenum, 1980).
74. Crain and Shen, After chronic opioid exposure sensory neurons become supersensitive to the excitatory effects of opioid agonists and antagonists as occurs after acute elevation of GM1 ganglioside. Brain Res., 575(1):13-24, 1992.

While the foregoing invention has been described in some detail for purposes of clarity and understanding, it will be appreciated by one skilled in the art, from a reading of the disclosure, that various changes in form and detail can be made without departing from the true scope of the invention in the appended claims.

## Claims

1. Use of a bimodally-acting opioid agonist and a GMi-ganglioside inhibitor for the manufacture of a medicament for treatment of pain.

2. The use of Claim 1, wherein the GM1-ganglioside inhibitor increases the analgesic potency of the bimodally-acting opioid agonist.

3. The use of Claim 1 or 2, wherein the GM1-ganglioside inhibitor attenuates an adverse excitatory effect of the bimodally-acting opioid agonist.

4. The use of Claim 3, wherein the adverse excitatory effect is selected from anti-analgesia, hyperalgesia, hyperexcitability, physical dependence, psychological dependence, opioid-mediated addiction, tolerance and withdrawal.

5. The use of Claim 4, wherein the adverse excitatory effect is selected from constipation, nausea, respiratory depression, sedation and vomiting.

6. The use of any of Claims 1-5, wherein the pain is selected from acute pain, chronic pain, nociceptive pain and neuropathic pain.

7. The use of any of Claims 1-6, wherein the bimodally-acting opioid agonist is selected from buprenorphine, butorphanol, codeine, dynorphins, endorphins, enkephalins, fentanyl analogues, hydromorphone, levorphanol, meperidine, methadone, morphine, nalbuphine, oxycodone, oxymorphone, pentazocine, propoxyphene, and tramadol.

8. The use of any of Claims 1-6, wherein the bimodally-acting opioid agonist is morphine.

9. The use of any of Claims 1-8, wherein the GM1-ganglioside inhibitor reduces GM1-ganglioside activity in nociceptive neurons by disabling, disrupting or inactivating the functions of GM1-ganglioside in nociceptive neurons, or by diminishing the levels or amount of GM1-ganglioside in nociceptive neurons.

10. The use of any of Claims 1-9, wherein the GM1-ganglioside inhibitor is selected from CTX-B, anti-GM 1-ganglioside antibody, oligonucleotide anti-sense to CTX-B, agents that decrease or inhibit cAMP, agents that decrease or inhibit glycosyltransferase, and neuraminidase inhibitors, including MgSO₄, Na₂SO₄, oseltamivir and zanamivir.

11. The use of any of Claims 1-9, wherein the GM1-ganglioside inhibitor is selected from CTX-B, oseltamivir, anti-GM 1-ganglioside antibody, and Na₂SO₄.

12. The use of any of Claims 1-9, wherein the GM1-ganglioside inhibitor is CTX-B or oseltamivir.

13. The use of any of Claims 1-9, wherein the GM1-ganglioside inhibitor is CTX-B.

14. The use of any of Claims 1-9, wherein the GM1-ganglioside inhibitor is oseltamivir.

15. The use of any of Claims 1-9, wherein the GM1-ganglioside inhibitor is Na₂SO₄.

16. The use of any of Claims 1-9, wherein the GM1-ganglioside inhibitor is an anti-GM1-ganglioside antibody.

17. The use of Claims 10-15, wherein the Na₂SO₄ is for administration in an amount of 10mg/kg, wherein the CTX-B is for administration in an amount of 0.01-1mg/kg, or wherein the oseltamivir is for administration in an amount of 0.1-1mg/kg.

18. The use of any of Claims 1-17, wherein the bimodally-acting opioid agonist and the GM1-ganglioside inhibitor are for oral administration, parenteral administration, nasal administration, sublingual administration, transdermal administration, or administration by liposomal delivery or osmotic pump.

19. The use of any of Claims 1-17, wherein the bimodally-acting opioid agonist and the GM1-ganglioside inhibitor are for nasal, oral, parenteral or transdermal administration.

20. The use of any of Claims 1-17, wherein the bimodally-acting opioid agonist and the GM1-ganglioside inhibitor are for nasal or oral administration.

21. A pharmaceutical composition comprising a bimodally-acting opioid agonist and a GM1-ganglioside inhibitor.

22. The pharmaceutical composition of Claim 21, wherein the composition is for treatment of pain.

23. The pharmaceutical composition of Claim 21 or 22, wherein the bimodally-acting opioid agonist is selected from buprenorphine, butorphanol, codeine, dynorphins, endorphins, enkephalins, fentanyl analogues, hydromorphone, levorphanol, meperidine, methadone, morphine, nalbuphine, oxycodone, oxymorphone, pentazocine, propoxyphene, and tramadol.

24. The pharmaceutical composition of Claim 21 or 22, wherein the bimodally-acting opioid agonist is morphine.

25. The pharmaceutical composition of any of Claims 21-24, wherein the GM1-ganglioside inhibitor reduces GM1-ganglioside activity in nociceptive neurons by disabling, disrupting or inactivating the functions of GM 1-ganglioside in nociceptive neurons, or by diminishing the levels or amount of GM1-ganglioside in nociceptive neurons.

26. The pharmaceutical composition of any of Claims 21-25, wherein the GM1-ganglioside inhibitor is selected from CTX-B, anti-GM 1-ganglioside antibody, oligonucleotide anti-sense to CTX-B, agents that decrease or inhibit cAMP, agents that decrease or inhibit glycosyltransferase, and neuraminidase inhibitors, including MgSO₄, Na₂SO₄, oseltamivir and zanamivir.

27. The pharmaceutical composition of any of Claims 21-25, wherein the GM1-ganglioside inhibitor is selected from CTX-B, oseltamivir, anti-GM 1-ganglioside antibody, and Na₂SO₄.

28. The pharmaceutical composition of any of Claims 21-25, wherein the GM1-ganglioside inhibitor is CTX-B or oseltamivir.

29. The pharmaceutical composition of any of Claims 21-25, wherein the GM1-ganglioside inhibitor is CTX-B.

30. The pharmaceutical composition of any of Claims 21-25, wherein the GM1-ganglioside inhibitor is oseltamivir.

31. The pharmaceutical composition of any of Claims 21-25, wherein the GM1-ganglioside inhibitor is Na₂SO₄.

32. The pharmaceutical composition of any of Claims 21-25, wherein the GM1-ganglioside inhibitor is an anti-GM1-ganglioside antibody.

33. The pharmaceutical composition of any of Claims 26-31, wherein the Na₂SO₄ is for-administration in an amount of 10mg/kg, wherein the CTX-B is for administration in an amount of 0.01-1mg/kg, or wherein the oseltamivir is for administration in an amount of 0.1-1mg/kg.

34. The pharmaceutical composition of any of Claims 21-33, for treating, reducing, inactivating, or attenuating an adverse excitatory effect associated with administration of the bimodally-acting opioid agonist.

35. The pharmaceutical composition of Claim 34, wherein the adverse excitatory effect is selected from anti-analgesia, hyperalgesia, hyperexcitability, physical dependence, psychological dependence, opioid-mediated addiction, tolerance and withdrawal.

36. The pharmaceutical composition of Claim 34, wherein the adverse excitatory effect is selected from constipation, nausea, respiratory depression, sedation and vomiting.

37. The pharmaceutical composition of any of Claims 22-36, wherein the pain is selected from acute pain, chronic pain, nociceptive pain and neuropathic pain.

38. The pharmaceutical composition of any of Claims 21-37, for oral administration, parenteral administration, nasal administration, sublingual administration, transdermal administration, or administration by liposomal delivery or osmotic pump.

39. The pharmaceutical composition of any of Claims 21-37, for nasal, oral, parenteral or transdermal administration.

40. The pharmaceutical composition of any of Claims 21-37, for nasal or oral administration.

## Patentansprüche

1. Verwendung eines bimodal wirkenden Opioidagonisten und eines GM1-Gangliosidinhibitors zur Herstellung eines Medikaments zur Schmerzbehandlung.

2. Verwendung gemäß Anspruch 1, wobei der GM1-Gangliosidinhibitor die schmerzlindernde Potenz des bimodal wirkenden Opioidagonisten erhöht.

3. Verwendung gemäß Anspruch 1 oder 2, wobei der GM1-Gangliosidinhibitor eine exzitatorische Nebenwirkung des bimodal wirkenden Opioidagonisten vermindert.

4. Verwendung gemäß Anspruch 3, wobei die exzitatorische Nebenwirkung ausgewählt ist aus Folgendem: Anti-Analgesie, Hyperalgesie, Hyperexzitabilität, physische Abhängigkeit, psychische Abhängigkeit, opioid-vermittelte Sucht, Toleranz und Entzug.

5. Verwendung gemäß Anspruch 4, wobei die exzitatorische Nebenwirkung ausgewählt ist aus Folgendem: Konstipation, Nausea, Atemdepression, Sedierung und Vomitus.

6. Verwendung gemäß einem der Ansprüche 1-5, wobei der Schmerz ausgewählt ist aus Folgendem: akuter Schmerz, chronischer Schmerz, nociceptiver Schmerz und neuropathischer Schmerz.

7. Verwendung gemäß einem der Ansprüche 1-6, wobei der bimodal wirkende Opioidagonist ausgewählt ist aus Folgendem: Buprenorphin, Butorphanol, Codein, Dynorphine, Endorphine, Enkephaline, Fentanyl-Analoge, Hydromorphon, Levorphanol, Meperidin, Methadon, Morphin, Nalbuphin, Oxycodon, Oxymorphon, Pentazocin, Propoxyphen und Tramadol.

8. Verwendung gemäß einem der Ansprüche 1-6, wobei der bimodal wirkende Opioidagonist Morphin ist.

9. Verwendung gemäß einem der Ansprüche 1-8, wobei der GM1-Gangliosidinhibitor die GM1-Gangliosidaktivität in nociceptiven Neuronen reduziert, indem er die Funktionen des GM1-Gangliosids in nociceptiven Neuronen unwirksam macht, unterbricht oder inaktiviert oder indem er die Konzentration oder die Menge des GM1-Gangliosids in nociceptiven Neuronen vermindert.

10. Verwendung gemäß einem der Ansprüche 1-9, wobei der GM1-Gangliosidinhibitor ausgewählt ist aus Folgendem: CTX-B, Anti-GM1-Gangliosidantikörper, Oligonucleotid-Antisense zu CTX-B, Wirkstoffe, die cAMP vermindern oder hemmen, Wirkstoffe, die Glykosyltransferase vermindern oder hemmen, sowie Neuraminidaseinhibitoren, einschließlich MgSO₄, Na₂SO₄, Oseltamivir und Zanamivir.

11. Verwendung gemäß einem der Ansprüche 1-9, wobei der GM1-Gangliosidinhibitor ausgewählt ist aus Folgendem: CTX-B, Oseltamivir, Anti-GM1-Gangliosidantikörper und Na₂SO₄.

12. Verwendung gemäß einem der Ansprüche 1-9, wobei der GM1-Gangliosidinhibitor CTX-B oder Oseltamivir ist.

13. Verwendung gemäß einem der Ansprüche 1-9, wobei der GM1-Gangliosidinhibitor CTX-B ist.

14. Verwendung gemäß einem der Ansprüche 1-9, wobei der GM1-Gangliosidinhibitor Oseltamivir ist.

15. Verwendung gemäß einem der Ansprüche 1-9, wobei der GM1-Gangliosidinhibitor Na₂SO₄ ist.

16. Verwendung gemäß einem der Ansprüche 1-9, wobei der GM1-Gangliosidinhibitor ein Anti-GM1-Gangliosidantikörper ist.

17. Verwendung gemäß den Ansprüchen 10-15, wobei das Na₂SO₄ zur Verabreichung in einer Menge von 10 mg/kg vorgesehen ist, wobei das CTX-B zur Verabreichung in einer Menge von 0,01-1 mg/kg vorgesehen ist oder wobei das Oseltamivir zur Verabreichung in einer Menge von 0,1-1 mg/kg vorgesehen ist.

18. Verwendung gemäß einem der Ansprüche 1-17, wobei der bimodal wirkende Opioidagonist und der GM1-Gangliosidinhibitor zur oralen Verabreichung, zur parenteralen Verabreichung, zur nasalen Verabreichung, zur sublingualen Verabreichung, zur transdermalen Verabreichung oder zur Verabreichung durch liposomale Applikation oder eine osmotische Pumpe vorgesehen sind.

19. Verwendung gemäß einem der Ansprüche 1-17, wobei der bimodal wirkende Opioidagonist und der GM1-Gangliosidinhibitor zur nasalen, oralen, parenteralen oder transdermalen Verabreichung vorgesehen sind.

20. Verwendung gemäß einem der Ansprüche 1-17, wobei der bimodal wirkende Opioidagonist und der GM1-Gangliosidinhibitor zur nasalen oder oralen Verabreichung vorgesehen sind.

21. Pharmazeutische Zusammensetzung, umfassend einen bimodal wirkenden Opioidagonisten und einen GM1-Gangliosidinhibitor.

22. Pharmazeutische Zusammensetzung gemäß Anspruch 21, wobei die Zusammensetzung für die Schmerzbehandlung vorgesehen ist.

23. Pharmazeutische Zusammensetzung gemäß Anspruch 21 oder 22, wobei der bimodal wirkende Opioidagonist ausgewählt ist aus Folgendem: Buprenorphin, Butorphanol, Codein, Dynorphine, Endorphine, Enkephaline, Fentanyl-Analoge, Hydromorphon, Levorphanol, Meperidin, Methadon, Morphin, Nalbuphin, Oxycodon, Oxymorphon, Pentazocin, Propoxyphen und Tramadol.

24. Pharmazeutische Zusammensetzung gemäß den Ansprüchen 21 oder 22, wobei der bimodal wirkende Opioidagonist Morphin ist.

25. Pharmazeutische Zusammensetzung gemäß einem der Ansprüche 21-24, wobei der GM1-Gangliosidinhibitor die GM1-Gangliosidaktivität in nociceptiven Neuronen reduziert, indem er die Funktionen des GM1-Gangliosids in nociceptiven Neuronen unwirksam macht, unterbricht oder inaktiviert oder indem er die Konzentration oder die Menge des GM1-Gangliosids in nociceptiven Neuronen vermindert.

26. Pharmazeutische Zusammensetzung gemäß einem der Ansprüche 21-25, wobei der GM1-Gangliosidinhibitor ausgewählt ist aus Folgendem: CTX-B, Anti-GMl-Gangliosidantikörper, Oligonucleotid-Antisense zu CTX-B, Wirkstoffe, die cAMP vermindern oder hemmen, Wirkstoffe, die Glykosyltransferase vermindern oder hemmen, sowie Neuraminidaseinhibitoren, einschließlich MgSO₄, Na₂SO₄, Oseltamivir und Zanamivir.

27. Pharmazeutische Zusammensetzung gemäß einem der Ansprüche 21-25, wobei der GM1-Gangliosidinhibitor ausgewählt ist aus Folgendem: CTX-B, Oseltamivir, Anti-GM1-Gangliosidantikörper und Na₂SO₄.

28. Pharmazeutische Zusammensetzung gemäß einem der Ansprüche 21-25, wobei der GM1-Gangliosidinhibitor CTX-B oder Oseltamivir ist.

29. Pharmazeutische Zusammensetzung gemäß einem der Ansprüche 21-25, wobei der GM1-Gangliosidinhibitor CTX-B ist.

30. Pharmazeutische Zusammensetzung gemäß einem der Ansprüche 21-25, wobei der GM1-Gangliosidinhibitor Oseltamivir ist.

31. Pharmazeutische Zusammensetzung gemäß einem der Ansprüche 21-25, wobei der GM1-Gangliosidinhibitor Na₂SO₄ ist.

32. Pharmazeutische Zusammensetzung gemäß einem der Ansprüche 21-25, wobei der GM1-Gangliosidinhibitor ein Anti-GM1-Gangliosidantikörper ist.

33. Pharmazeutische Zusammensetzung gemäß einem der Ansprüche 26-31, wobei das Na₂SO₄ zur Verabreichung in einer Menge von 10 mg/kg vorgesehen ist, wobei das CTX-B zur Verabreichung in einer Menge von 0,01-1 mg/kg vorgesehen ist oder wobei das Oseltamivir zur Verabreichung in einer Menge von 0,1-1 mg/kg vorgesehen ist.

34. Pharmazeutische Zusammensetzung gemäß einem der Ansprüche 21-33 zur Behandlung, Reduzierung, Inaktivierung oder Verminderung einer exzitatorischen Nebenwirkung des bimodal wirkenden Opioidagonisten.

35. Pharmazeutische Zusammensetzung gemäß Anspruch 34, wobei die exzitatorische Nebenwirkung ausgewählt ist aus Folgendem: Anti-Analgesie, Hyperalgesie, Hyperexzitabilität, physische Abhängigkeit, psychische Abhängigkeit, opioid-vermittelte Sucht, Toleranz und Entzug.

36. Pharmazeutische Zusammensetzung gemäß Anspruch 34, wobei die exzitatorische Nebenwirkung ausgewählt ist aus Folgendem: Konstipation, Nausea, Atemdepression, Sedierung und Vomitus.

37. Pharmazeutische Zusammensetzung gemäß einem der Ansprüche 22-36, wobei der Schmerz ausgewählt ist aus Folgendem: akuter Schmerz, chronischer Schmerz, nociceptiver Schmerz und neuropathischer Schmerz.

38. Pharmazeutische Zusammensetzung gemäß einem der Ansprüche 21-37, die zur oralen Verabreichung, zur parenteralen Verabreichung, zur nasalen Verabreichung, zur sublingualen Verabreichung, zur transdermalen Verabreichung oder zur Verabreichung durch liposomale Applikation oder eine osmotische Pumpe vorgesehen ist.

39. Pharmazeutische Zusammensetzung gemäß einem der Ansprüche 21-37, die zur nasalen, oralen, parenteralen oder transdermalen Verabreichung vorgesehen ist.

40. Pharmazeutische Zusammensetzung gemäß einem der Ansprüche 21-37, die zur nasalen oder oralen Verabreichung vorgesehen ist.

## Revendications

1. Utilisation d'un agoniste opioïde à action bimodale et d'un inhibiteur des gangliosides GM1 pour la fabrication d'un médicament pour le traitement de la douleur.

2. L'utilisation de la revendication 1, où l'inhibiteur des gangliosides GM1 accroît la puissance analgésique de l'agoniste opioïde à action bimodale.

3. L'utilisation de la revendication 1 ou 2, où l'inhibiteur des gangliosides GM1 atténue un effet excitateur indésirable de l'agoniste opioïde à action bimodale.

4. L'utilisation de la revendication 3, où l'effet excitateur indésirable est sélectionné parmi anti-analgésie, hyperalgésie, hyperexcitabilité, dépendance physique, dépendance psychologique, accoutumance par l'intermédiaire d'opioïdes, tolérance et manque.

5. L'utilisation de la revendication 4, où l'effet excitateur indésirable est sélectionné parmi constipation, nausée, dépression respiratoire, sédation et vomissement.

6. L'utilisation de n'importe lesquelles des revendications 1 à 5, où la douleur est sélectionnée parmi douleur aiguë, douleur chronique, douleur nociceptive et douleur neuropathique.

7. L'utilisation de n'importe lesquelles des revendications 1 à 6, où l'agoniste opioïde à action bimodale est sélectionné parmi buprénorphine, butorphanol, codéine, dynorphines, endorphines, encéphalines, analogues du fentanyl, hydromorphone, lévorphanol, mépéridine, méthadone, morphine, nalbuphine, oxycodone, oxymorphone, pentazocine, propoxyphène, et tramadol.

8. L'utilisation de n'importe lesquelles des revendications 1 à 6, où l'agoniste opioïde à action bimodale est de la morphine.

9. L'utilisation de n'importe lesquelles des revendications 1 à 8, où l'inhibiteur des gangliosides GM1 réduit l'activité des gangliosides GM1 dans les neurones nociceptifs en neutralisant, interrompant ou inactivant les fonctions des gangliosides GM1 dans les neurones nociceptifs, ou en diminuant les niveaux ou la quantité de gangliosides GM1 dans les neurones nociceptifs.

10. L'utilisation de n'importe lesquelles des revendications 1 à 9, où l'inhibiteur des gangliosides GM1 est sélectionné parmi CTX-B, anticorps anti-gangliosides GM1, oligonucléotide antisens à la CTX-B, agents qui réduisent ou inhibent l'AMPc, agents qui réduisent ou inhibent la glycosyltransférase, et inhibiteurs de la neuraminidase, y compris MgSO₄, Na₂SO_{4,} oseltamivir et zanamivir.

11. L'utilisation de n'importe lesquelles des revendications 1 à 9, où l'inhibiteur des gangliosides GM1 est sélectionné parmi CTX-B, oseltamivir, anticorps anti-gangliosides GM1, etNa₂SO_{4.}

12. L'utilisation de n'importe lesquelles des revendications 1 à 9, où l'inhibiteur des gangliosides GM1 est CTX-B ou oseltamivir.

13. L'utilisation de n'importe lesquelles des revendications 1 à 9, où l'inhibiteur des gangliosides GM1 est CTX-B.

14. L'utilisation de n'importe lesquelles des revendications 1 à 9, où l'inhibiteur des gangliosides GM1 est oseltamivir.

15. L'utilisation de n'importe lesquelles des revendications 1 à 9, où l'inhibiteur des gangliosides GM1 est Na₂SO₄.

16. L'utilisation de n'importe lesquelles des revendications 1 à 9, où l'inhibiteur des gangliosides GM1 est un anticorps anti-gangliosides GM1.

17. L'utilisation des revendications 10 à 15, où le Na₂SO₄ est destiné à être administré dans une quantité de 10 mg/kg, où la CTX-B est destinée à être administrée dans une quantité de 0,01 à 1 mg/kg, ou où l'oseltamivir est destiné à être administré dans une quantité de 0,1 à 1 mg/kg.

18. L'utilisation de n'importe lesquelles des revendications 1 à 17, où l'agoniste opioïde à action bimodale et l'inhibiteur des gangliosides GM1 sont destinés à une administration orale, une administration parentérale, une administration nasale, une administration sublinguale, une administration transdermique, ou une administration par libération liposomale ou pompe osmotique.

19. L'utilisation de n'importe lesquelles des revendications 1 à 17, où l'agoniste opioïde à action bimodale et l'inhibiteur des gangliosides GM1 sont destinés à une administration nasale, orale, parentérale ou transdermique.

20. L'utilisation de n'importe lesquelles des revendications 1 à 17, où l'agoniste opioïde à action bimodale et l'inhibiteur des gangliosides GM1 sont destinés à une administration nasale ou orale.

21. Une composition pharmaceutique comportant un agoniste opioïde à action bimodale et un inhibiteur des gangliosides GM1.

22. La composition pharmaceutique de la revendication 21, où la composition est pour le traitement de la douleur.

23. La composition pharmaceutique de la revendication 21 ou 22, où l'agoniste opioïde à action bimodale est sélectionné parmi buprénorphine, butorphanol, codéine, dynorphines, endorphines, encéphalines, analogues du fentanyl, hydromorphone, lévorphanol, mépéridine, méthadone, morphine, nalbuphine, oxycodone, oxymorphone, pentazocine, propoxyphène, et tramadol.

24. La composition pharmaceutique de la revendication 21 ou 22, où l'agoniste opioïde à action bimodale est de la morphine.

25. La composition pharmaceutique de n'importe lesquelles des revendications 21 à 24, où l'inhibiteur des gangliosides GM1 réduit l'activité des gangliosides GM1 dans les neurones nociceptifs en neutralisant, interrompant ou inactivant les fonctions des gangliosides GM1 dans les neurones nociceptifs, ou en diminuant les niveaux ou la quantité de gangliosides GM1 dans les neurones nociceptifs.

26. La composition pharmaceutique de n'importe lesquelles des revendications 21 à 25, où l'inhibiteur des gangliosides GM1 est sélectionné parmi CTX-B, anticorps anti-gangliosides GM1, oligonucléotide antisens à la CTX-B, agents qui réduisent ou inhibent l'AMPc, agents qui réduisent ou inhibent la glycosyltransférase, et inhibiteurs de la neuraminidase, y compris MgSO₄, Na₂SO₄, oseltamivir et zanamivir.

27. La composition pharmaceutique de n'importe lesquelles des revendications 21 à 25, où l'inhibiteur des gangliosides GM1 est sélectionné parmi CTX-B, oseltamivir, anticorps anti-gangliosides GM1, et Na₂SO₄.

28. La composition pharmaceutique de n'importe lesquelles des revendications 21 à 25, où l'inhibiteur des gangliosides GM1 est CTX-B ou oseltamivir.

29. La composition pharmaceutique de n'importe lesquelles des revendications 21 à 25, où l'inhibiteur des gangliosides GM1 est CTX-B.

30. La composition pharmaceutique de n'importe lesquelles des revendications 21 à 25, où l'inhibiteur des gangliosides GM1 est oseltamivir.

31. La composition pharmaceutique de n'importe lesquelles des revendications 21 à 25, où l'inhibiteur des gangliosides GM1 est Na₂SO₄.

32. La composition pharmaceutique de n'importe lesquelles des revendications 21 à 25, où l'inhibiteur des gangliosides GM1 est un anticorps anti-gangliosides GM1.

33. La composition pharmaceutique de n'importe lesquelles des revendications 26 à 31, où le Na₂SO₄ est destiné à être administré dans une quantité de 10 mg/kg, où la CTX-B est destinée à être administrée dans une quantité de 0,01 à 1 mg/kg, ou où l'oseltamivir est destiné à être administré dans une quantité de 0,1 à 1 mg/kg.

34. La composition pharmaceutique de n'importe lesquelles des revendications 21 à 33, pour traiter, réduire, inactiver, ou atténuer un effet excitateur indésirable associé à l'administration de l'agoniste opioïde à action bimodale.

35. La composition pharmaceutique de la revendication 34, où l'effet excitateur indésirable est sélectionné parmi anti-analgésie, hyperalgésie, hyperexcitabilité, dépendance physique, dépendance psychologique, accoutumance par l'intermédiaire d'opioïdes, tolérance et manque.

36. La composition pharmaceutique de la revendication 34, où l'effet excitateur indésirable est sélectionné parmi constipation, nausée, dépression respiratoire, sédation et vomissement.

37. La composition pharmaceutique de n'importe lesquelles des revendications 22 à 36, où la douleur est sélectionnée parmi douleur aiguë, douleur chronique, douleur nociceptive et douleur neuropathique.

38. La composition pharmaceutique de n'importe lesquelles des revendications 21 à 37, pour une administration orale, une administration parentérale, une administration nasale, une administration sublinguale, une administration transdermique, ou une administration par libération liposomale ou pompe osmotique.

39. La composition pharmaceutique de n'importe lesquelles des revendications 21 à 37, pour une administration nasale, orale, parentérale ou transdermique.

40. La composition pharmaceutique de n'importe lesquelles des revendications 21 à 37, pour une administration nasale ou orale.
